# EUROPEAN PATENT SPECIFICATION

(11) **EP 4 017 291 B1**
(45) Date of publication and mention of the grant of the patent: **27.11.2024**
(21) Application number: 20760833.2
(22) Date of filing: 20.08.2020
(51) Int. Cl.: A24B 3/14, A24B 15/12, A61M 11/04, A61M 15/06, A24D 1/20, A24D 3/17

(54) **A SUBSTITUTE SMOKING CONSUMABLE**
RAUCHERSATZVERBRAUCHSMITTEL
CONSOMMABLE DE SUBSTITUTION DU TABAC

(30) Priority: 23.08.2019 EP 19193283; 23.08.2019 EP 19193275; 23.08.2019 EP 19193272; 23.08.2019 EP 19193270; 23.08.2019 EP 19193264; 23.08.2019 EP 19193259; 23.08.2019 EP 19193279
(43) Date of publication of application: 29.06.2022
(73) Proprietor: Imperial Tobacco Limited, Bristol, BS3 2LL (GB)
(72) Inventor: MURRAY, Samantha, Liverpool Merseyside L24 9HP (GB); MISTRY, Hushmita, Liverpool Merseyside L24 9HP (GB); LORD, Chris, Liverpool Merseyside L24 9HP (GB); FERRIE, Kate, Liverpool Merseyside L24 9HP (GB); SHENTON, Ross, Liverpool Merseyside L24 9HP (GB); RIETH, Fabian, Liverpool Merseyside L24 9HP (GB); GUMMERSON, Siobhan, Liverpool Merseyside L24 9HP (GB); JONES, Stephen, Liverpool Merseyside L24 9HP (GB); STUART, Ian, Liverpool Merseyside L24 9HP (GB)
(74) Representative: Mewburn Ellis LLP
(86) International application number: PCT/EP2020/073438
(87) International publication number: WO 2021/037694

(56) References cited:
- EP-A1- 2 907 397
- WO-A1-2013/022936
- WO-A1-2017/072146
- WO-A1-2017/202965
- WO-A1-2018/122375
- CN-A- 106 136 305
- US-A1- 2018 077 967

## Description

### Field of the disclosure

The present disclosure relates to a consumable for a smoking substitute device. In particular, but not exclusively, to a heat not burn consumable. It also relates to a heat not burn system comprising a consumable and a heating element, and a device for housing the system.

### Background

The "smoking" of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful by-products. There have been proposed various smoking substitute systems (or "substitute smoking systems") in order to avoid the smoking of tobacco.

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or flavourings without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products. Some smoking substitute systems use smoking substitute articles that are designed to resemble a traditional cigarette and are cylindrical in form with a mouthpiece at one end.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories. A known smoking substitute system is described in US2018077967A1.

There are a number of different categories of smoking substitute systems, each utilising a different smoking substitute approach.

One approach for a smoking substitute system is the so-called "heat not burn" ("HNB") approach in which tobacco (rather than an "e-liquid") is heated or warmed to release vapour. The tobacco may be leaf tobacco or reconstituted tobacco. The vapour may contain nicotine and/or flavourings. In the HNB approach the intention is that the tobacco is heated but not burned, i.e. the tobacco does not undergo combustion.

A typical HNB smoking substitute system may include a device and a consumable. The consumable may include the tobacco material. The device and consumable may be configured to be physically coupled together. In use, heat may be imparted to the tobacco material by a heating element of the device, wherein airflow through the tobacco material causes moisture in the tobacco material to be released as vapour. A vapour may also be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the consumable (entrained in the airflow) from an inlet to a mouthpiece (outlet), the vapour cools and condenses to form an aerosol for inhalation by the user. The aerosol will normally contain the volatile compounds.

In HNB smoking substitute systems, heating as opposed to burning the tobacco material is believed to cause fewer, or smaller quantities, of the more harmful compounds ordinarily produced during smoking. Consequently, the HNB approach may reduce the odour and/or health risks that can arise through the burning, combustion and pyrolytic degradation of tobacco.

A first existing implementation of the HNB approach is the IQOS^{™} device from Philip Morris Ltd. The IQOS^{™} device uses a consumable, including reconstituted tobacco contained within a metallic foil and paper wrapper. The consumable is a cylindrical, rod-shaped consumable designed to resemble a traditional cigarette which is inserted into a heater device. The heater device has a thermally conductive heating blade which penetrates the reconstituted tobacco of the consumable, when the consumable is inserted into the heating device. Activation of the heating device heats the heating element, which, in turn, heats the tobacco in the consumable. The heating of the tobacco causes it to release nicotine vapour and flavours which may be drawn through the mouthpiece by the user through inhalation.

A second existing implementation of the HNB approach is the device known as Glo^{™} from British American Tobacco. Glo^{™} also uses a rod-shaped consumable similar in appearance to a traditional cigarette. The consumable includes reconstituted tobacco in a paper wrapping which is heated in a heating device. When the consumable is placed in the heating device, the tobacco is surrounded by a heating element. Activation of the heating device heats the heating element, which, in turn, heats the tobacco in the consumable. The heating of the tobacco causes it to release nicotine vapour and flavours which may be drawn through the consumable by the user through inhalation. The tobacco, when heated by the heating device, is configured to produce vapour when heated rather than when burned (as in a traditional cigarette). The tobacco may contain high levels of aerosol formers (carrier), such as vegetable glycerine ("VG") or propylene glycol ("PG").

Common to both the IQOS^{™} and Glo^{™} systems is uneven and incomplete heating of the tobacco, or possible burning of some regions of the tobacco.

Both devices also fail to conceal the residues which remain in the consumable after heating, these residues being both aesthetically unpleasing and also presenting a contamination risk to the user during removal of the consumable from the device.

Furthermore, the aerosol formers may leach from the consumable to stain and/or dampen the paper wrapping which is aesthetically unappealing and which can lead to transfer of the aerosol formers to contaminate the user.

Aspects and embodiments of the disclosure were devised with the foregoing in mind.

### Summary

At its most general, the present disclosure relates to an aerosol-forming article e.g. a smoking substitute article such as an HNB consumable comprising an aerosol-forming substrate and a downstream filter having upper and lower surfaces spaced by longitudinally-extending transverse surfaces wherein at least one of the surfaces is a curved or rounded surface or comprises a curved or rounded surface portion and wherein the filter has a non-circular transverse cross section.

In a first aspect, there is provided a heat not burn (HNB) consumable comprising an aerosol-forming substrate and a downstream filter having upper and lower surfaces spaced by opposing longitudinally-extending transverse surfaces wherein the filter has a greater width (between the transverse surfaces) than depth (between the upper and lower surfaces) and wherein the filter comprises at least one inwardly-extending air flow path extending from at least one of the upper, lower or transverse surfaces into the filter, characterised in that the at least one air flow path is defined by a channel having an opening provided on one of the surfaces.

By providing an air flow path into the filter from one of the surfaces, air can be drawn into the aerosol-forming substrate as the user inhales and this air can help to cool and mix the vapour. The cross-sectional area of the at least one air flow path and/or the number of radial air flow paths can be tailored to tailor the resistance to draw (RTD) of the consumable.

Optional features will now be set out. These are applicable singly or in any combination with any aspect.

As used herein, the terms "upstream" and "downstream" are intended to refer to the flow direction of the vapour/aerosol i.e. with the downstream end of the consumable being the mouth end or outlet where the aerosol exits the consumable for inhalation by the user. The upstream end of the consumable is the opposing end to the downstream end. The terms "upper" and "lower" as used herein are not intended to infer any orientation of the substrate/consumable before, during or after use.

There may be a plurality of air flow paths spaced e.g. equally spaced around the perimeter of the filter. The plurality of air flow paths may be axially aligned with one another e.g. axially aligned and equally spaced around the perimeter of the filter. The axially aligned air flow paths may be axially spaced by a substantially equal amount from the upstream and downstream axial end of the filter.

There may be two or more groups (which may be axially spaced from one another) of axially aligned (e.g. axially aligned, equally spaced) air flow paths. The or each air flow path may extend towards (e.g. may extend to) the axial centre of the filter.

In some embodiments, the upper and lower surfaces of the filter are substantially planar and may be equally spaced by the transverse surfaces (i.e. the upper and lower surfaces are parallel to one another) such that the filter is a planar filter.

The opposing transverse surfaces may be planar and substantially parallel to one another. Where the upper and lower surfaces are planar, the planar transverse surfaces may be substantially perpendicular to the upper and lower surfaces such that the planar filter has a substantially rectangular transverse cross section i.e. the filter is a cuboid filter.

The transverse cross section is defined by a face having edges defining the width and depth i.e. the term "transverse cross section" is used to denote a cross section through the consumable perpendicular to the longitudinal axis/length of the planar filter/consumable. The filter has opposing longitudinal end faces (an upstream end face and a downstream end face) which will each comprise a transverse cross section.

In some embodiments, at least one of the upper, lower and transverse surfaces is a curved or rounded surface or comprises a curved or rounded surface portion.

By providing a filter having at least one (e.g. opposing transverse surfaces) which are curved or have a curved portion, the filter can be designed to better receive aerosol/vapour from a correspondingly shaped substrate. Such a filter can also be housed within a correspondingly-shaped housing along with the substrate to minimise any unfilled volume within the housing thus minimising the size of the housing design.

In some embodiments, the upper and lower surfaces of the filter are substantially planar and may be equally spaced by the transverse surfaces (i.e. the upper and lower surfaces are parallel to one another) such that the filter is a planar filter.

In some embodiments, the planar filter has at least one and preferably two curved or rounded (concave or convex) opposing transverse surfaces.

For example, one or both of the opposing transverse surfaces may comprise a substantially convex surface (e.g. a semi-circular surface) such that the filter has a substantially obround transverse cross section i.e. the filter is an obround cylindrical filter.

In some embodiments, one or both of the opposing transverse surfaces may be concave or may comprise one or more concave portions. For example, the or each curved/rounded opposing transverse surface(s) may each comprise longitudinally-extending upper and lower concave portions which meet at a longitudinally-extending ridge.

The concave portion(s) may be spaced from the planar upper and lower surfaces by opposing convex portions such that the transverse cross-section is a modified obround where the opposing side edges of the cross-section each take the form of a curly brace/bracket i.e. "{" and "}". Hereinafter, such a filter will be referred to as a "modified obround cylindrical filter".

In other embodiments, the opposing transverse surfaces may be planar or as described above (i.e. convex, concave or convex and concave) and one or both of the upper/lower surfaces may be curved/rounded e.g. they may be convex rounded surfaces. Where the upper and lower surfaces are convex surfaces and the transverse surfaces are planar, the filter may have a truncated oval transverse cross-section. Where the upper and lower surfaces are convex surfaces and the transverse surfaces are convex, the filter may have an oval transverse cross-section. Where the upper and lower surfaces are convex surfaces and the transverse surfaces comprise two concave portions meeting at a longitudinally extending ridge, the filter may have a modified mandorla transverse cross-section.

The filter of the first aspect preferably has a greater width and length than depth. The depth of the filter may be between 4 and 8 mm, e.g. between 5 and 7 mm e.g. around 6 mm. The width of the filter may be between 7 and 18 mm e.g. between 8 and 14 mm or 10 and 12 mm. The length of the filter may be between 2mm and 25 mm e.g. between 3mm and 22mm.

The filter of the first aspect has an upstream longitudinal end face which faces and may abut the downstream longitudinal end face of the substrate. The downstream longitudinal end face of the filter of the first aspect may comprises a curved/rounded surface (e.g. a convex surface such as a semi-circular surface).

The filter of the first aspect may comprise a hollow bore. The hollow bore may extend from the upstream longitudinal end face of the filter to the downstream longitudinal face of the filter.

The hollow bore may have a circular, rectangular or obround transverse cross sectional area. The bore may have a uniform transverse cross-sectional area. The transverse cross-section of the hollow bore may have the same shape as the transverse cross-section of the filter. Where the filter of the first aspect comprises at least one air flow path, it/they may extend to the hollow bore.

The filter of the first aspect may be comprised of cellulose acetate or polypropylene tow. The filter may be comprised of activated charcoal. The filter may be comprised of paper. The filter may be comprised of plant material e.g. extruded or pressed plant material. The filter of the first aspect may be circumscribed with a plug wrap e.g. a paper plug wrap. Where the filter of the first aspect comprises at least one air flow path, the plug wrap may comprise at least one aperture aligned with the at least one air flow path.

For the avoidance of doubt, the filter may have a density/porosity/permeability that at least partly blocks the passage (filters out) at least one of the components of the aerosol/vapour or, in other embodiments, the "filter" may have a density/porosity/permeability such that it is permeable to (allows the passage of) all components of the aerosol/vapour.

In some embodiments, the filter of the first aspect may comprise at least one liquid release member.

The liquid release member can comprise an envelope for containing the liquid. The envelope can be rigid and fragmentable under pressure (e.g. upon contact with the heating element). Alternatively, the envelope can be meltable upon application of heat. The liquid release member may contain an aerosol former such as vegetable glycerine and/or propylene glycol. By containing the aerosol former within a liquid release member that is configured to release the liquid (e.g. aerosol former) upon use, seepage of the liquid from the consumable to contaminate the user is avoided. The liquid release member may comprise a flavouring.

The liquid release member may positioned proximal the abutment between the filter (e.g. at the upstream longitudinal end face of the filter) and the substrate (i.e. the downstream longitudinal end face of the filter) so that upon release, the liquid can penetrate the plant product in the substrate.

The filter described above in the first aspect can be combined with any of the aspects described below.

The aerosol-forming substrate is capable of being heated to release at least one volatile compound that can form an aerosol.

The discussion below referring to the aerosol-forming substrate is applicable to all aspects of the invention.

The aerosol-forming substrate may be located at the upstream end of the consumable. The transverse cross-section of the filter of the first aspect may match the transverse cross-section of the substrate.

The substrate in any of the aspects may comprise upper and lower surfaces spaced by opposing longitudinally-extending transverse surfaces wherein the depth of the substrate (between the upper and lower surfaces) and the width of the substrate (between the opposing transverse surfaces) are unequal e.g. the width is greater than the depth.

In some embodiments, the upper and lower surfaces are substantially planar and may be equally spaced by the transverse surfaces (i.e. the upper and lower surfaces are parallel to one another) such that the substrate is a planar substrate.

By providing the substrate as a planar substrate rather than as a cylindrical rod (having a substantially circular cross section), the substrate has a greater exposed surface area for contact with a heating element thus allowing quicker and more even heat transfer from the heating element to the plant product. In this manner, heating of the substrate can be effected using a heating element at a lower temperature (e.g. around 250 °C) which reduces the chances of burning of the plant product.

The opposing transverse surfaces may be planar and substantially parallel to one another. Where the upper and lower surfaces are planar, the planar transverse surfaces may be substantially perpendicular to the upper and lower surfaces such that the planar substrate has a substantially rectangular transverse cross section i.e. the substrate is a cuboid substrate.

The transverse cross section is defined by a face having edges defining the width and depth i.e. the term "transverse cross section" is used to denote a cross section through the consumable perpendicular to the longitudinal axis/length of the planar substrate/consumable. The substrate has opposing longitudinal end faces (an upstream end face and a downstream end face) which will each comprise a transverse cross section.

In some embodiments, the substrate has at least one curved or rounded surface but a non-circular transverse cross section.

Accordingly, the present disclosure also relates to an aerosol-forming article e.g. a smoking substitute article such as an HNB consumable comprising an aerosol-forming substrate having upper and lower surfaces spaced by longitudinally-extending transverse surfaces wherein at least one of the surfaces is a curved or rounded surface or comprises a curved or rounded surface portion and wherein the substrate has a non-circular transverse cross section.

In a third aspect, there is provided a heat not burn (HNB) consumable comprising an aerosol-forming substrate having upper and lower surfaces spaced by opposing longitudinally-extending transverse surfaces wherein at least one of the surfaces is a curved or rounded surface or comprises a curved or rounded surface portion and wherein the substrate has a greater width (between the transverse surfaces) than depth (between the upper and lower surfaces).

Providing a substrate having a greater width than depth, rather than a substrate comprising a cylindrical rod (having a substantially circular cross section), allows the substrate to abut or receive a planar heating element aligned with the width direction. Thus there is a greater surface area for contact with the heating element thus allowing quicker and more even heat transfer from the heating element to the substrate.

In preferred embodiments of the third aspect, the substrate is an extruded substrate and the one or both of the opposing transverse surfaces comprises a concave surface/surface portion.

In a fourth aspect, there is provided a heat not burn (HNB) consumable comprising an aerosol-forming substrate having upper and lower surfaces spaced by opposing longitudinally-extending transverse surfaces wherein the substrate has a width between the transverse surfaces, a depth between the upper and lower surfaces and a length perpendicular to the width and depth, wherein:
a) the aspect ratio of the width to the length is between 1:1 and 1:5;
b) the aspect ratio of the width to the depth is greater than 1:1 such that the width is greater than the depth; and
c) the aspect ratio of the length to the depth is greater than 1:1 such that the length is greater than the depth; and
d) at least one of the upper, lower or transverse surfaces is a curved or rounded surface or comprises a curved or rounded surface portion.

Providing a substrate having a greater length than depth allows the substrate to take an elongated form that is convenient for gripping by the user for insertion into and removal from a heat not burn device. The combination of the greater width and length provides a greater surface area to abut or receive a planar heating element aligned with the width direction. Thus there is a greater surface area for contact with the heating element thus allowing quicker and more even heat transfer from the heating element to the substrate. Providing a smaller depth (than width and length) allows rapid heating throughout the depth of the substrate.

In some embodiments of the fourth aspect, at least one of the opposing transverse surfaces is a concave surface or comprises one or more concave portions.

In some embodiments of the fourth aspect, the aspect ratio of the width to the length is between 1:1 and 1:3 e.g. between 1:1 and 1:2.5 such as between 1:1 and 1:2, for example, between 1:1 and 1:1.5. In some embodiments, the aspect ratio of the width to the length is less than 1:1 (such that the width is less than the length). In some embodiments of the fourth aspect, the aspect ratio of the width to the depth is between 1:0.05 and 1:0.9 such as between 1:0.1 and 1:0.8 or between 1:0.2 and 1:0.6 e.g. around 1:0.5 or 1:0.6.ln some embodiments of the fourth aspect, the aspect ratio of the length to the depth is between 1:0.05 and 1:0.8 such as between 1:0.1 and 1:0.7 or between 1:0.3 and 1:0.6, e.g. between 1:0.4 and 1:0.6.

Where the consumable has a non-uniform depth or width as a result of the curved surface(s) (portion(s)), references to the width and depth are intended to refer to the maximum width/depth.

Accordingly, for the third and fourth aspects, heating of the substrate can be effected using a heating element at a lower temperature (e.g. around 250 °C) which reduces the chances of burning of the substrate. The curved surface(s) (portion(s)) reduces the number of (or eliminates) right-angled corners associated with a planar substrate having a rectangular transverse cross section. Such corners present areas of substrate that are remote from the heating element and thus prone to incomplete heating. For example, in any of the aspects disclosed herein, at least one and preferably both of the opposing transverse surfaces of the substrate may comprise a curved or rounded surface/surface portion e.g. at least one and preferably both of the opposing transverse surfaces comprises a convex or concave surface/surface portion.

For example, one or both of the opposing transverse surfaces of the substrate may comprise a substantially convex surface (e.g. a semi-circular surface). Accordingly the planar substrate has a substantially obround transverse cross section i.e. the substrate is an obround cylindrical substrate.

In some embodiments, one or both of the opposing transverse surfaces of the substrate may be concave or may comprise one or more concave portions. For example, the or each curved/rounded opposing transverse surface(s) may each comprise longitudinally-extending upper and lower concave portions which meet at a longitudinally-extending ridge.

The concave portion(s) may be spaced from the planar upper and lower surfaces by opposing convex portions such that the transverse cross-section is a modified obround where the opposing side edges of the cross-section each take the form of a curly brace/bracket i.e. "{" and "}". Hereinafter, such a substrate will be referred to as a "modified obround cylindrical substrate".

In other embodiments, the opposing transverse surfaces of the substrate may be as described above (i.e. planar, convex, concave or convex and concave) and one or both of the upper/lower surfaces may be curved/rounded e.g. they may be convex rounded surfaces. Where the upper and lower surfaces are convex surfaces and the transverse surfaces are convex, the substrate may have an oval transverse cross-section. Where the upper and lower surfaces are convex surfaces and the transverse surfaces are planar, the substrate may have a truncated oval transverse cross-section. Where the upper and lower surfaces are convex surfaces and the transverse surfaces comprise two concave portions meeting at a longitudinally extending ridge, the substrate may have a modified mandorla transverse cross-section.

The substrate of any aspect preferably has a greater width and length than depth. The length and width may be equal but, preferably, the length is greater than the width such that the substrate has substantially rectangular upper and lower surfaces. The length of the substrate (between the upstream and downstream end faces) may be between 10 and 20 mm e.g. between 10 and 15 mm. The width of the substrate (between opposing transverse surfaces) may be between 7 and 18 mm e.g. between 8 and 14 mm or 10 and 12 mm. The depth of the substrate (between the upper and lower surfaces) may be between 1 and 8 mm, e.g. between 2 and 7 mm e.g. around 2 mm or around 6 mm.

In some embodiments, the consumable comprises a single substrate e.g. a single planar substrate as described above. In this case, the depth of the substrate is preferably between 5 and 7 mm e.g. around 6 mm.

The substrate may have a single heating surface (one of the upper and lower surfaces) for contact with/for facing a heating element (e.g. a planar heating element) or there may be two opposing surfaces (both of the upper and lower surfaces) each for contact with/for facing one of two heating elements (e.g. planar heating elements). The plant product is then heated externally and inwards from the upper and/or lower heating surfaces.

In preferred embodiments, there is a constant depth of plant product between the surface that is heated and the opposing surface. Thus the substrate comprises a heating surface (e.g. a substantially planar heating surface) which, in use, faces the heating element (e.g. a planar heating element), and at least one opposing surface, wherein the depth of the plant product between the heating surface and the at least one and opposing surface is substantially constant.

By providing the substrate with a substantially planar heating surface (for thermal contact with a heating element), the plant product has a greater exposed surface area for contact with a heating element for allowing quicker heating. The constant depth of plant product between the surfaces results in more even heat transfer from the heating element to the plant product. In this manner, heating of the plant product can be effected using a heating element at a lower temperature (e.g. around 250 °C) which reduces the chances of burning of the plant product.

The depth of the plant product between the heating and opposing surfaces may be between 1 and 8 mm, e.g. between 2 and 7 mm e.g. around 2 mm or around 6 mm.

In other embodiments, the substrate is heated internally and outwards (towards the upper and lower surfaces). This may be achieved by providing a penetrable substrate such that a heating element can be inserted into the substrate e.g. into the upstream end face of the substrate.

Alternatively, the substrate may have a hollow core for releasably and slidably receiving the heating element. In such an embodiment, air may flow through the hollow core and through the (porous) body of the substrate. In such an embodiment, the airflow downstream of the substrate may be a combination of airflow through the substrate and airflow through the hollow core of the substrate.

In use, the hollow core receives a heating element (i.e. by insertion of the heating element into the hollow core) which can contact the internal surfaces defining the core thus allowing quicker and more even heat transfer from the heating element to the plant product. In this manner, heating of the plant product can be effected using a heating element at a lower temperature (e.g. around 250 °C) which reduces the chances of burning of the plant product.

The hollow core is defined by a longitudinally-extending recess extending from the upstream end face of the substrate. The core recess may extend from the upstream end face to the opposing downstream end face.

The core recess is defined by upper and lower inner surfaces spaced by opposing longitudinally extending inner transverse surfaces. The upper and lower inner surfaces will face the heating element in use. The depth of the core recess (between the upper and lower inner surfaces) and the width of the recess (between the opposing inner transverse surfaces) are unequal.

In some embodiments, the upper and lower inner surfaces are substantially planar and may be equally spaced by the inner transverse surfaces (i.e. the upper and lower inner surfaces are parallel to one another).

The opposing inner transverse surfaces may be substantially parallel to one another and substantially perpendicular to the upper and lower inner surfaces such that the core recess has a substantially rectangular transverse cross section i.e. the core recess is a cuboid core recess.

In other embodiments, at least one and preferably both of the opposing inner transverse surfaces may comprise a curved or rounded (concave or convex) surface. For example, one or both of the opposing inner transverse surfaces may comprise a substantially convex surface (e.g. a semi-circular surface) such that the core recess has a substantially obround transverse cross section i.e. the core recess is an obround core recess.

Where the substrate is a hollow cuboid substrate, it may comprise a cuboid core recess. Where the substrate is an obround cylindrical substrate or a modified obround cylindrical substrate, it may comprise an obround core recess.

The shape of the transverse cross section of the core recess may match the shape of the transverse cross section of the substrate.

The recess may have a depth (between the upper and lower inner surfaces) of between 0.5 and 2 mm e.g. around 1 mm. The recess may have a width (between the opposing inner transverse surfaces) of between 7 and 14 mm e.g. between 7 and 12 mm or 8 and 10 mm e.g. around 8 mm. The length of the recess may be between 10 and 20 mm e.g. between 10 and 15 mm. In these embodiments, the depth of the hollow substrate may be between 4 and 8 mm, e.g. between 5 and 7 mm e.g. around 6 mm.

In other embodiments, the consumable comprises a plurality of substrates e.g. two planar substrates (which may each be as described above). Where there are two planar substrates, the depth of each planar substrate is preferably between 1 and 8 mm, e.g. between 2 and 5 mm e.g. around 2 mm.

The planar substrates are preferably aligned and spaced from one another to define a planar recess therebetween such that the consumable has a substantially rectangular transverse cross section.

A heating element can be inserted into the planar recess so as to be releasably housed in the recess. In this way, heat can be transferred quickly and evenly to the plant product via the surfaces defining the planar recess.

In these embodiments, the substrates will each having an inner heating surface facing the planar recess and an opposing outer surface. The two planar substrates are preferably vertically and horizontally aligned. The planar recess is also vertically and horizontally aligned with the planar substrates.

The surfaces defining the core recess or planar recess may be lined with a thermally conductive material. For example, the surface(s) defining the recess may be at least 50% or 60% covered and preferably at least 70 % or 80% or 90% covered. The recess may be fully lined with the thermally conductive material. The thermally conductive material may be provided as a foil which may be textured e.g. dimpled.

The substrate may comprise at least one channel extending into the plant product from either or both of the upstream and downstream longitudinal end faces of the substrate. The thermally conductive material may extend into the at least one channel. For example, the thermally conductive material may extend from the recess to the at least one channel over the upstream/downstream longitudinal end face of the substrate. This helps increase heat transfer from the heating element within the recess into the substrate.

The consumable may comprise a further layer of the thermally conductive material, or of a further thermally conductive material, on an outer surface of the plant product opposing the recess.

The thermally conductive material or the further thermally conductive material may be selected from the group consisting of: carbon or metal/metal alloy such as aluminium; brass; copper; gold; steel; silver; an alloy of one of more thereof; or a mixture of two or more thereof.

The substrate may be dosed with an e-liquid either in its entirety or in selected portions. For example, the substrate may be dosed with e-liquid at or proximal its heating surfaces. The substrate may be dosed with e-liquid at its surfaces which face the heating element(s). For example, the substrate may be dosed with e-liquid at or proximal its upper and/or lower surfaces.

Where the substrate is a hollow substrate and comprises a hollow core defined by a core recess, the plant product at or proximal one or more of the upper/lower/transverse inner surfaces defining the core recess may be dosed with e-liquid.

Where the consumable comprises a plurality of planar substrates defining a planar recess, the plant product at or proximal one or both of the surfaces of the planar substrates facing the planar recess may be dosed with e-liquid. The e-liquid may contain aerosol formers such as polyglycol (PG) and/or vegetable glycerine (VG). It may contain flavourings.

The substrate may comprise a hydrophobic or liquid-impermeable outer coating (e.g. on at least the upper and lower surfaces) to prevent seepage or transfer of the e-liquid from the substrate.

The consumable of any aspect (e.g. the third or fourth aspect) may further comprise a filter as described above for the first and second aspect.

In some embodiments, the aspect ratio of the width to the length of the filter is between 1:1 and 1:3.4, such as between 1:1 and 3:1 or between 1:1 and 1:2.5, for example, between 1:1.5 and 1:2.2. In some embodiments, the aspect ratio of the width to the depth is between 1:0.2 and 1:0.9 such as between 1:0.3 and 1:0.9 or between 1:0.8 and 1:0.8, for example between 1:0.4 and 1:0.7. In some embodiments, the aspect ratio of the length to the depth is between 1:0.1 and 1:0.8 such as between 1: 0.2 and 1:0.7 or between 1:0.2 and 1:0.6, for example, around 1:0.3.

The consumable of any aspect may comprise a spacer e.g. a paper/cardboard spacer interposed between the filter and the substrate. The spacer defines a space or cavity or chamber downstream from the aerosol-forming substrate. For example, it may be provided between the aerosol-forming substrate and the filter. The spacer acts to allow both cooling and mixing of the aerosol.

The spacer may be a planar spacer e.g. having a substantially rectangular or substantially obround transverse cross section. The spacer may have a transverse cross-section matching the transverse cross section of the substrate and/or filter.

The spacer preferably has a greater width and length than depth. The length and width may be equal but, preferably, the width is greater than the length. The depth of the spacer may be between 4 and 8 mm, e.g. between 5 and 7 mm e.g. around 6 mm. The width of the spacer may be between 7 and 18 mm e.g. between 8 and 14 mm or 10 and 12 mm.

The consumable of any aspect may further comprise a wrapping e.g. a paper or cardboard wrapping that encloses the upper and lower surfaces and the transverse walls of the substrate and filter (and spacer where present).

In embodiments where the substrate comprises at least one channel extending into the plant product from the upstream longitudinal end face of the substrate (as described above), the wrapping e.g. the cardboard wrapping may comprise a transverse extension which extends to cover a portion of the upstream longitudinal end face of the substrate. The transverse extension may then comprise an inwardly-depending axial extension extending inwards into the at least one channel in the substrate.

The consumable of any aspect may comprise a housing i.e. the substrate may be at least partly (and preferably entirely) enclosed within the housing. The housing of any of the first to fourth aspects may be as described below (e.g. as described for any one or more of the fifth to seventh aspects.

The housing may have a non-circular transverse cross-section. The transverse cross-section of the housing may match the transverse cross-section of the substrate.

The present disclosure also relates to an aerosol-forming article e.g. a smoking substitute article such as an HNB consumable comprising a housing for at least partly containing an aerosol-forming substrate, the housing having an end wall with one or more apertures formed therein for airflow through the substrate.

In a fifth aspect, there is provided a heat not burn (HNB) consumable having a housing comprising: an outlet aperture at a downstream end of the housing; an end wall at an opposing upstream end of the housing, the end wall comprising at least one inlet aperture formed therein; and a chamber housing an aerosol forming substrate, the chamber fluidly connected between the at least one inlet aperture and the outlet aperture.

The provision of a housing e.g. a housing having a chamber that houses the aerosol forming substrate may e.g. protect the aerosol forming substrate from the external environment. The provision of an inlet aperture may allow airflow through or past the aerosol forming substrate during use.

The end wall may be at a longitudinal end of the housing. In this respect, the end wall may be an upstream longitudinal end wall of the housing. In other embodiments, the end wall may be integral with the rest of the housing. Hence, the end wall may be formed of the same material as the rest of the housing.

The at least one aperture may be spaced from a centre (i.e. central point) or central region of the end wall (e.g. the membrane). The at least one aperture may be located closer to a periphery of the end wall than the centre (i.e. central point) of the end wall. The housing may be elongate so as to define a longitudinal axis extending centrally through the housing. The centre of the end wall may be aligned with the longitudinal axis of the housing.

The at least one aperture may be configured (e.g. sized and shaped) so as to substantially prevent material of the aerosol forming substrate from passing therethrough. For example, where the aerosol forming substrate is formed of e.g. shreds or granules, the at least one aperture may be sized such that it has at least one dimension (e.g. width, length, diameter) smaller than the shreds or granules. In this way, the end wall may retain the aerosol forming substrate in the chamber, whilst the at least one aperture allows the flow of fluid (i.e. air) into and through the chamber. The at least one aperture may be circular, or e.g. may be a slot. The at least one aperture may have a diameter (or width, or length) that is less than e.g. 3 mm, or less than e.g. 2 mm or 1 mm.

The end wall may comprise at least two apertures. The end wall may comprise more than two apertures (e.g. three, four, or five apertures). The at least two aperture may both be spaced from the centre (or central region) of the end wall. The at least two apertures may be spaced either side of the centre of the end wall. For example, the at least two apertures and the centre of the end wall may be aligned in a linear manner (i.e. along an axis perpendicular to the longitudinal axis of the housing).

In preferred embodiments, the end wall comprises two inlet apertures, the two inlet apertures laterally spaced either side of the centre of the end wall.

The provision of a plurality of apertures may further provide a pressure drop within the aerosol forming substrate, so as to reduce the speed of the air flowing through the aerosol-forming substrate. This may increase the quantity of vapour/aerosol entrained in the air flow in use.

The housing may have a non-circular transverse cross-section. The transverse cross-section of the housing may match the transverse cross-section of the substrate. The transverse cross-section of the housing may match the shape of the end wall.

The present disclosure also relates to an aerosol-forming article, e.g. a smoking substitute article such as a smoking substitute consumable, having a housing configured to direct aerosol to a mouth of a user.

In a sixth aspect, there is provided a heat not burn (HNB) consumable comprising a housing defining a chamber having an upstream portion housing an aerosol-forming substrate and a downstream chimney portion, wherein the chimney portion is tapered for directing aerosol from the substrate towards a downstream aperture.

In this way, aerosol released from the aerosol-forming substrate may be directed towards the downstream aperture, and therefore the mouth of a user, by the housing of the HNB consumable itself. Accordingly, the flow of aerosol through the consumable may be improved. Furthermore, the number of components of the HNB consumable, and thus its complexity, may be reduced.

The substrate is a solid substrate (as opposed to a liquid (e.g. e-liquid) substrate).

The chimney portion may extend from an upstream end to the downstream aperture in a longitudinal (axial) direction of the consumable. The chimney portion may be axially aligned with the longitudinal axis of the consumable.

A transverse cross-sectional area of the chimney portion (i.e. the cross sectional area perpendicular to the longitudinal axis of the chimney/consumable) may reduce towards the downstream aperture. In this way, the chimney portion is tapered towards the downstream aperture, such that aerosol released from the aerosol-forming substrate travels through the chimney portion and is directed towards the downstream aperture.

A transverse cross-sectional area of the upstream end of the chimney portion may be greater than a transverse cross-sectional area of the downstream aperture. The transverse cross-sectional area of the chimney portion may reduce continuously from its upstream end to the downstream aperture. The transverse cross-sectional area of the upstream portion of the chamber may be substantially constant along the length (in a longitudinal axial direction) of the upstream portion.

The transverse cross-sectional shape of the chimney portion may be uniform along the entire length of the chimney portion (wherein the length of the chimney portion is aligned with the longitudinal axis of the consumable). For example, the chimney portion may have a rectangular, oval, obround, circular, square along its entire length.

The chimney portion may extend from the upstream portion of the chamber housing the substrate to the downstream aperture. In this embodiment, the upstream end of the chimney portion may be adjacent to (e.g. may abut) the substrate.

The features of the housing defined in the fifth and sixth aspects may be combined with each other and may be combined with any other aspect either separately or in combination.

The downstream aperture may be formed in a downstream longitudinal end wall of the housing. In some embodiments, the downstream aperture may be centred in the downstream longitudinal end wall (i.e. the downstream aperture may be longitudinally aligned with a central axis of the consumable). The housing may comprise an opposing, at least partly open, upstream end face. Alternatively, the housing may have an upstream end wall that at least partly (and preferably fully) obscures the substrate from view.

The housing may have a single heating surface (an outer surface of one of the upper and lower walls) for contact with/for facing a heating element (e.g. a planar heating element) or there may be two opposing surfaces (outer surfaces of both of the upper and lower walls) each for contact with/for facing one of two heating elements (e.g. planar heating elements). The housing, and thus the aerosol-forming substrate contained in the housing, is then heated externally and inwards from the heating surface(s) of the upper and/or lower walls.

The inner surfaces of the upper, lower and transverse walls of an upstream portion of the housing may define the upstream portion of the chamber.

In other embodiments, the housing may comprise an inner sleeve which lines the housing walls (as discussed further below) and an upstream portion of the inner sleeve may define the upstream portion of the chamber. The upstream portion of the inner sleeve may conform to the shape of the walls of the upstream portion of the housing i.e. it may define upper and lower upstream inner surfaces spaced by opposing longitudinally-extending transverse upstream inner surfaces.

Accordingly, the upstream portion of the chamber may have a transverse cross-sectional shape matching the transverse cross-sectional shape of the housing i.e. it may have a substantially rectangular, (modified) obround, oval, truncated oval, or a modified mandorla transverse cross-section.

The upstream portion of the chamber may be defined by a textured inner surface (on the inner surface of the housing walls or on the inner sleeve), e.g. it may have a mesh texture.

The chimney portion of the chamber may be partly defined by the inner surfaces of the upper and lower walls of a downstream portion of the housing.

In other embodiments where the housing comprises an inner sleeve which lines the housing walls, a downstream portion of the inner sleeve may define the chimney portion of the chamber. The downstream portion of the inner sleeve may comprise upper and lower downstream inner surfaces spaced by opposing longitudinally-extending downstream transverse inner surfaces.

The depth of the chimney portion (i.e. the spacing between the inner surfaces of the upper and lower walls of the downstream portion of the housing or the spacing between the upper and lower downstream surfaces of the inner sleeve) may be substantially constant along the length of the chimney portion. The inner surfaces of the upper and lower walls of the downstream portion of the housing may be planar and substantially parallel to one another or the upper and lower downstream surfaces of the inner sleeve may be planar and substantially parallel to one another.

In other embodiments, the depth of the chimney portion may reduce towards the downstream aperture.

The chimney portion of the chamber may be partly defined by longitudinally-extending transverse chimney walls. The transverse chimney walls may extend (in a depth direction perpendicular to the longitudinal axis of the consumable) between the inner surfaces of the upper and lower walls of the downstream portion of the housing or, where the housing comprises an inner sleeve, the transverse chimney walls may form part of the downstream portion of the inner sleeve extending between the upper and lower downstream surfaces of the inner sleeve.

In embodiments in which the transverse cross-sectional area of the chimney portion reduces towards the downstream aperture, the transverse spacing (width) between the transverse chimney walls defining the chimney portion may reduce towards the downstream aperture. In this way, the width of the chimney portion (between the transverse chimney walls) reduces towards the downstream aperture. The width of the chimney portion may reduce continuously from the upstream end of the chimney portion to the downstream aperture.

The transverse chimney walls may each comprise a substantially planar surface facing the chimney portion. In other embodiments, the transverse chimney walls may comprise a substantially convex surface facing the chimney portion.

The angle formed between each transverse chimney wall and its respective transverse housing wall at the upstream end of the chimney portion may be equal to one another. Specifically, this angle may be in the range of 1° to 89°, more preferably 1° to 45°, more preferably 5° to 30°. At the downstream end of the chimney portion, the transverse chimney walls may extend substantially parallel to the transverse walls of the housing.

In some embodiments, the longitudinally-extending transverse chimney walls may be formed by webbing within the housing. Specifically, the webbing may comprise two webs, each web extending from an inner surface of a respective transverse wall of the housing transversely inwards towards the axial (longitudinal) centre of the housing and longitudinally to the downstream longitudinal end wall of the housing.

Each web may be nonlinear. Specifically, each web may curve inwardly (i.e. in the transverse direction, into the consumable) from its respective transverse wall of the housing towards the opposing web, and longitudinally to the downstream aperture. The angle formed between each web and its respective transverse wall at the upstream end of the chimney portion may be equal to one another. Specifically, this angle may be in the range of 1° to 89°, more preferably 1° to 45°, more preferably 5° to 30°. At the downstream end of the chimney portion, the webs may extend substantially parallel to the transverse walls. Accordingly, the downstream end of each web is spaced from its respective transverse wall.

Preferably, each web may have a wall thickness in the range of 0.8 to 8.0 mm, e.g. 1.5 to 5.0 mm. Each web may be formed at least partly and preferably entirely of a biodegradable material such as cornstarch, bamboo, wood, palm, sugarcane, cardboard or paperboard, recycled or recyclable (thermoplastic) polymer material.

In embodiments comprising the webbing described above, the chimney portion is further defined by the inner surfaces of the upper and lower walls of the housing as described above. Accordingly, the webbing spaces the upper and lower walls of the housing, and is substantially perpendicular to the upper and lower walls of the housing.

The webbing may be integrally formed with the walls of the housing such that the upper and lower walls, the transverse walls, and the webbing (transverse chimney walls) may be formed or moulded from a single housing component. In this way, the number of components of the HNB consumable may be reduced.

Alternatively, each of the webs may be attached to the inner surfaces of the upper and lower walls, longitudinal downstream end wall and/or respective transverse wall, for example by an adhesive such as a biodegradable glue.

As discussed above, in some embodiments, the housing comprises an inner sleeve which lines the walls of the housing. Accordingly, the inner sleeve may comprise upper and lower walls spaced by opposing longitudinally-extending transverse walls. The inner surfaces of the inner sleeve define the chamber.

The walls of the housing may substantially surround and enclose the inner sleeve. The inner sleeve may be attached to, and therefore fixed within, the housing walls, for example, by an adhesive such as biodegradable glue.

As discussed above, the inner sleeve may comprise an upstream portion which substantially conforms to the shape of the upstream portion of the housing i.e. the upstream portion of the housing and the upstream portion of the inner sleeve have substantially the same transverse cross-sectional shape. Accordingly, the inner sleeve and housing may be contiguous in the upstream portion (i.e. in the portion defining the upstream portion of the chamber).

In the downstream portion of the housing, the inner sleeve may not be contiguous with the housing. The transverse walls of the inner sleeve (which will define the transverse chimney walls in the chimney portion) may be transversely spaced from the inner surface of the transverse housing walls.

Specifically, each transverse wall of the inner sleeve in the downstream portion is deflected inwardly (i.e. in the transverse direction, into the consumable) from the respective transverse wall of the housing towards the opposing transverse wall of the inner sleeve.

Preferably, the inner sleeve has a thickness such that the combined thickness of the housing walls and inner sleeve is in the range of 0.8 to 8.0 mm, e.g. 1.5 to 5.0 mm.

The inner sleeve may be formed at least partly and preferably entirely of a biodegradable material such as cornstarch, bamboo, wood, palm, sugarcane, cardboard or paperboard, recycled or recyclable (thermoplastic) polymer material.

The consumable of the sixth aspect may further comprise a downstream element downstream of the substrate. The downstream element may be positioned upstream or downstream of the chimney portion, or alternatively, the downstream element may be positioned within the chimney portion itself.

The downstream element may have a non-circular transverse cross-section. The transverse cross-section of the downstream element may match the transverse cross-section of the substrate. The downstream element may have any of the transverse cross-sectional shapes described above for the substrate.

The downstream element may be a porous element. The porous element may have a porosity such that it at least partly blocks the passage (filters out) at least one of the components of the aerosol/vapour. Thus the downstream porous element may be a filter element as described above for the first and second aspect. In other embodiments, the porous element may have a density/porosity/permeability such that it is permeable to (allows the passage of) all components of the aerosol/vapour.

In yet further embodiments, the downstream element may be a solid element having a hollow bore for the passage of the aerosol/vapour.

The housing (of any aspect e.g. of any of the first to sixth aspects) may be self-supporting. The term "self-supporting" is intended to refer to a housing formed of a material that does not flex or bend under its own weight.

Preferably, the housing is formed of a material that is substantially rigid or semi-rigid i.e. it is not easily flexible.

The paper wrappers provided on the prior art consumables are relatively thin and flimsy. Whilst physically containing the plant product before and after use of the consumable, they do not effectively contain residues in the spent consumable and handling of the spent consumable can result in residue transfer to the user. By providing a more structurally robust (self-supporting) housing, the consumable becomes more akin to a cartridge or "pod" that effectively contains residue after use to protect a user from contamination. At least a portion and preferably the whole of the housing has a wall thickness in the range of 0.8 to 8.0 mm, e.g. 1.5 to 5.0 mm.

The housing may have an inner surface defining the chamber housing the substrate. The inner surface may be textured e.g. it may have a mesh texture.

The housing may be formed at least partly and preferably entirely of a biodegradable material such as cornstarch, bamboo, wood, palm, sugarcane, cardboard or paperboard, recycled or recyclable (thermoplastic) polymer material.

It may be formed of moulded pulp material e.g. natural fibre pulp material. The housing may be at least partly formed of moulded tobacco cellulose pulp, wood pulp, bamboo pump, palm pulp or bagasse pulp. Bagasse pulp is most preferred.

The housing may comprise upper and lower walls spaced by opposing longitudinally-extending transverse walls wherein the depth of the housing (between the upper and lower walls) and the width of the housing (between the opposing transverse walls) are unequal e.g. the width is greater than the depth.

The housing may have a substantially constant transverse cross-sectional area.

The end wall may comprise opposing upper and lower edges, and transverse edges extending between the upper and lower edges. The upper and lower edges may be substantially linear (i.e. straight) and parallel to one another. The end wall may comprise at least one curved edge portion (e.g. convex or concave edge portion). For example, at least one and preferably both of the opposing transverse edges may be a curved. The concave edge portion(s) may be spaced from the upper and edges by opposing convex edge portions such that the shape of the end wall is a modified obround where the opposing transverse edges of the shape each take the form of a curly brace/bracket i.e. "{" and "}".

The length (between the upper and lower edges) and width (between the opposing transverse edges) of the end wall may be unequal e.g. the width may be greater than the length. In such an embodiment, the at least one aperture may be equally spaced from the upper and lower edges. For example, the end wall may comprise two apertures spaced equally between the upper and lower edges. Each of the two apertures may be located proximate a respective transverse edge of the end wall.

As should be appreciated, the opening of the housing (sealed by the end wall) may have a shape that is similar to (or the same as) the shape of the end wall as described above (e.g. the opening may have a modified obround shape). The opening may be configured (e.g. sized and/or shaped) for receipt of the substrate therethrough (i.e. into the chamber), for example, during assembly of the consumable.

The housing may have a depth of between 6 and 13 mm e.g. between 7 and 12 mm. The housing may have a width of between 9 and 23mm, e.g. 10 and 19 mm such as between 12 and 17 mm. The housing may have a length greater than 20mm. It may have a length of up to 45mm, or 42mm or 40 mm or 37mm.

In some embodiments the aspect ratio of the width to the length of the housing is between 1:1 and 1:5, such as between 1:1 and 1:4.5 or between 1:1 and 1:3.8, for example, between 1:2 and 1:3.

In some embodiments, the aspect ratio of the width to the depth of the housing is between 1:0.2 and 1:0.9 such as between 1:0.3 and 1:0.8 or between 1:0.4 and 1:0.8, for example between 1:0.4 and 1:0.7.

In some embodiments, the aspect ratio of the length to the depth of the housing is between 1:0.1 and 1:0.7 such as between 1: 0.2 and 1:0.6 or between 1:0.2 and 1:0.4, for example, around 1:0.3.

In some embodiments (of any aspect), the upper and lower walls of the housing are substantially planar and may be equally spaced by the transverse walls (i.e. the upper and lower walls are parallel to one another) such that the housing is a planar housing.

The opposing transverse walls of the housing may be planar and substantially parallel to one another. Where the upper and lower walls are planar, the planar transverse walls may be substantially perpendicular to the upper and lower walls such that the planar housing has a substantially rectangular transverse cross section i.e. the housing is a cuboid housing.

In some embodiments, the housing has at least one curved or rounded wall (e.g. a concave or convex wall) but a non-circular transverse cross section.

For example, at least one and preferably both of the opposing transverse walls may be a curved or rounded wall (e.g. a concave or convex wall).

For example, one or both of the opposing transverse walls may be a substantially convex wall (e.g. a semi-circular wall). Accordingly the planar housing may have a substantially obround transverse cross section i.e. the housing is an obround cylindrical housing.

In some embodiments, one or both of the opposing transverse walls may be a concave wall or may comprise one or more concave portions. For example, the or each curved/rounded opposing transverse wall(s) may each comprise longitudinally-extending upper and lower concave portions which meet at a longitudinally-extending ridge.

The concave portion(s) may be spaced from the planar upper and lower walls by opposing convex portions such that the transverse cross-section is a modified obround where the opposing side edges of the cross-section each take the form of a curly brace/bracket i.e. "{" and "}". Hereinafter, such a housing will be referred to as a "modified obround cylindrical housing".

In other embodiments, the opposing transverse walls may be as described above (i.e. planar, convex, concave or convex and concave) and one or both of the upper/lower walls may be curved/rounded e.g. they may be convex rounded walls. Where the upper and lower walls are convex walls and the transverse walls are convex, the housing may have an oval transverse cross-section. Where the upper and lower walls are convex walls and the transverse walls are planar, the housing may have a truncated oval transverse cross-section. Where the upper and lower walls are convex walls and the transverse walls comprise two concave portions meeting at a longitudinally extending ridge, the housing may have a modified mandorla transverse cross-section.

The chamber within and defined by the inner surfaces of the housing walls may be a cuboid chamber, an obround cylindrical chamber, a modified obround cylindrical chamber or a modified mandorla chamber.

The chamber within the housing preferably has the same transverse cross section as the housing.

Preferably, the transverse cross-section of the housing and the chamber matches the transverse cross-section of the substrate.

The housing may have an at least partly open upstream longitudinal end face. Alternatively, as discussed below (and as defined for the fifth aspect), the upstream longitudinal end face may comprise an upstream end wall that at least partly (and preferably fully) obscures the substrate from view. The housing may substantially fully enclose the aerosol forming substrate (i.e. except for the aperture formed in the end wall in the fifth aspect). In this way, the housing may obscure the aerosol forming substrate from view.

The housing may have an opposing downstream longitudinal end wall. The downstream longitudinal end wall may comprise at least one outlet/mouthpiece aperture. The downstream longitudinal end wall may comprise a curved/rounded (e.g. a convex/semi-circular) end wall.

The inner surface of the downstream longitudinal end wall of the housing may abut the downstream longitudinal end surface of the filter.

At least one (and optionally both) of the opposing transverse walls of the housing may comprise a longitudinally-extending junction such that the housing can be opened to expose the chamber within.

The downstream longitudinal end /wall may also comprise a junction.

For example, both of the opposing transverse walls and the downstream longitudinal end wall could comprise a respective junction such that the housing can be split into two opposing parts allowing for easy insertion during manufacture of the substrate (and filter/spacer where present).

Alternatively, one of the opposing transverse walls and the upstream longitudinal end wall may contain the junctions and the other transverse wall may contain a longitudinally extending hinge portion such that the housing may be opened along the junctions by pivoting of the two opposing parts about the hinge portion. Where the consumable comprises two planar substrates, each planar substrate may be mounted (e.g. glued) into a respective part (e.g. half) of the housing such that when the two opposing parts are brought together, the planar substrates are spaced from one another to define the planar recess therebetween (as discussed above).

The walls(s) comprising a junction may each comprise an affixing portion e.g. respective laterally extending flanges on either side of the junction that face each other to provide an increased surface area for fixing the opposing parts together.

The opposing housing parts may be attached together (e.g. between respective flanges), for example by an adhesive such as a biodegradable glue.

In embodiments comprising features of the sixth aspect in which the longitudinally-extending transverse chimney walls are formed by webbing, each of the two webs spacing the upper and lower walls of the housing may comprise a junction. In this way, each web can be split into two opposing webbing parts, which, when the consumable is assembled, align to define the chimney portion.

In embodiments comprising an inner sleeve within the housing, the inner sleeve may comprise two opposing sleeve parts which, when the consumable is assembled, are brought together to define the downstream chimney portion of the chamber (and optionally to define the upstream portion of the chamber).

Each part of the inner sleeve may comprise an affixing portion e.g. respective laterally extending flanges that face each other to provide an increased surface area for fixing the opposing sleeve parts together.

The two opposing sleeve parts may be attached together (e.g. between respective flanges extending at least partly around each sleeve part), for example by an adhesive such as a biodegradable glue.

Where the housing is split into two opposing parts and the substrate comprises two planar substrates, each of the two planar substrates may be mounted (e.g. glued) into a respective part (e.g. half) of the housing such that when the two opposing parts are brought together, the planar substrates are spaced from one another to define the planar recess therebetween (as discussed above).

As discussed above, the downstream longitudinal end of the housing comprises a downstream longitudinal end wall. The outlet aperture (e.g. defined in the fifth aspect) may be formed in this downstream longitudinal end of the housing. The longitudinal end wall (and the outlet aperture) may define a mouthpiece of the consumable (i.e. for receipt in a user's mouth).

The filter is typically provided adjacent e.g. with its downstream longitudinal end face abutting this longitudinal end wall of the housing (i.e. adjacent the outlet). Thus the downstream longitudinal end wall at least partly (and preferably completely) obscures/conceals the filter from view by the user.

By concealing the filter from view, the user is not exposed to the residues remaining in the filter/consumable after use thus improving the aesthetic appeal of the consumable after use and avoiding transfer of residue to the user.

Although the downstream longitudinal end wall may comprise one or more outlet(s)/mouthpiece aperture(s), this/these are typically small enough that visual inspection of the filter is significantly impeded compared to the prior art consumable where the end face of the filter is completely exposed. Thus whilst the downstream longitudinal wall may be discontinuous, it preferably covers (e.g. overlies or abuts) at least 20% e.g. at least 30 or 40 % and preferably at least 50%, e.g. at least 70% such as at least 80% or 90% of the surface area of the downstream longitudinal end face of the filter.

Similarly, the upstream longitudinal end face may comprise the/an upstream longitudinal end wall that at least partly obscures the substrate from view at least prior to use.

The upstream longitudinal end face of the housing (of any aspect) may comprise an upstream longitudinal end wall for at least partly overlying (e.g. abutting) the upstream longitudinal end face of the substrate. The upstream longitudinal end wall may comprise an aperture (into which the heating element can be inserted).

The upstream longitudinal end wall may be a perimeter wall i.e. it may extend only around one or more of the edges of the upstream longitudinal end face of the housing. For example, it may extend around all edges to form a frame defining the aperture (into which the heating element can be inserted). The aperture may be dimensioned to match the dimensions of the hollow core recess when the substrate is a hollow core substrate.

In other embodiments, the upper longitudinal end wall of the housing may extend along the upper and lower edges to form rails defining the aperture therebetween. The aperture may be dimensioned to match the dimensions of the planar recess when the consumable comprises two planar substrates.

In embodiments where the substrate comprises at least one channel extending into the plant product from the upstream longitudinal end face of the plant product (as described above), the upstream longitudinal end wall may comprise an inwardly-depending axial extension, extending inwards into the at least one channel in the substrate.

The upstream longitudinal end face of the housing may additionally or alternatively comprise a pierceable or peelable membrane such as a metallic foil or plastic membrane. Accordingly, in the fifth aspect, the end wall may be in the form of a pierceable membrane or may comprise a pierceable membrane. The membrane may be mounted across the entire open upstream longitudinal end face of the housing or it may be mounted on the upstream longitudinal end wall across the aperture. The membrane seals the upstream longitudinal end face prior to use and is pierced to mount the consumable on the heating element.

The membrane may seal the opening prior to use and may be pierced to mount the consumable on a heating element of a heat not burn device. The provision of at least one aperture in the pierceable membrane (in addition to a piercing created upon insertion of the heating element) may allow air to flow through the membrane and into the cavity containing the aerosol forming substrate even when a heating element is received therethrough. This may help to thermally manage the aerosol-forming substrate when e.g. it is heated by a heating element. That is, the air may help to distribute heat throughout the aerosol-forming substrate so as to avoid hot spots within the aerosol forming substrate in use.

When the end wall is (or comprises) a pierceable membrane, the pierceable membrane may extend fully across the opening so as to substantially seal the opening (i.e. except for the at least one aperture extending therethrough). Thus, the pierceable membrane may define an outer surface of the consumable. The pierceable seal may be formed of a foil, such as a metallic (e.g. aluminium) or plastic foil. The periphery (e.g. a peripheral edge) of the pierceable seal may be attached to the housing. The pierceable seal may be adhered to the housing (i.e. by an adhesive). The pierceable seal may be configured to be pierceable by way a heating element of a heat not burn device.

The consumable e.g. the consumable of the fifth aspect may further comprise a filter downstream of the substrate. The filter may have a non-circular transverse cross-section. The transverse cross-section of the filter may match the transverse cross-section of the substrate. The filter may be positioned between the downstream end of the substrate and the outlet of the housing. The filter may be as described for the first/second aspects.

The present disclosure also relates to an aerosol-forming article e.g. a smoking substitute article such as an HNB consumable comprising a moisture resistant surface.

In a seventh aspect, there is provided a heat not burn (HNB) consumable comprising an aerosol-forming substrate housed within a moulded or extruded housing, the housing having an inner surface facing the substrate and an opposing outer surface wherein at least a portion of at least one of the inner and outer surfaces of the housing comprises a moisture resistant surface.

By providing a moisture resistant surface (e.g. a liquid impermeable surface) on the inner and/or outer surface of the housing, leaching of any liquid components of the aerosol-forming substrate through the housing to soil the user is prevented.

In some embodiments, the moisture resistant surface may comprise a hydrophobic coating i.e. at least a portion of at least one of the inner and outer surfaces of the housing may comprise a hydrophobic coating. The hydrophobic coating (especially when provided on the exterior of the housing) is preferably a food grade hydrophobic coating. The hydrophobic coating is preferably a biodegradable hydrophobic coating. The hydrophobic coating may be a wax such as bees wax or carnauba wax.

In other embodiments, the moisture resistant surface may be textured to provide hydrophobic properties e.g. the surface may have a three-dimensional surface structure providing nano- or micrometre scale roughness. The hydrophobic surface protuberances have been found to reduce the effective surface contact area with moisture.

The term "hydrophobic" is used to describe a coating/surface that provides a water contact angle of greater than 70 degrees e.g. greater than 90 degrees at 25°C. In some embodiments, the hydrophobic coating/surface may have a contact angle greater than 120 or 130 or 140 or 150 degrees at 25°C.

In some embodiments, the moisture resistant surface comprises a layer of material (e.g. a layer of biodegradable/plant material such as natural fibre pulp material) having a greater density (lower porosity) than the housing such that the reduced porosity physically limits moisture penetration. For example, the housing and denser moisture resistant layer may comprise the same (plant) material, with the moisture resistant layer having a reduced porosity. The denser moisture resistant layer may have a porosity of less than 20% e.g. less than 10% or less than 5 % or 2% or 1% (where porosity is a measure of void volume to total volume and may be determined using CT scanning or a gas expansion method).

The hydrophobic coating/surface or denser moisture resistant layer may be provided on the inner surface e.g. on the entire inner surface of the housing.

The hydrophobic coating/surface or denser moisture resistant layer may be provided on the outer surface of the housing.

The housing may comprise an outlet/mouthpiece aperture formed at a downstream lateral end of the housing e.g. in a downstream longitudinal end wall of the housing. The hydrophobic coating/surface or denser moisture resistant layer may be provided on the outer surface of the housing surrounding the mouthpiece aperture. It has been found that providing a hydrophobic coating/surface on the exterior of the housing proximal the mouthpiece aperture prevents any adherence of the housing to the user's mouth.

The hydrophobic coating/surface or denser moisture resistant layer may be provided on the entire outer surface of the housing.

As discussed above, the moisture resistant layer may be formed of the same material as the housing but having a greater density (reduced porosity). Accordingly, the denser moisture resistant layer may be formed of a biodegradable material such as cornstarch, bamboo, wood, palm, sugarcane, cardboard or paperboard, recycled or recyclable (thermoplastic) polymer material.

It may be formed of moulded pulp material e.g. natural fibre pulp material. The denser moisture resistant layer may be at least partly formed of moulded tobacco cellulose pulp, wood pulp, bamboo pump, palm pulp or bagasse pulp. Bagasse pulp is most preferred.

In order to generate an aerosol, the substrate (in any aspect) comprises at least one volatile compound that is intended to be vaporised/aerosolised and that may provide the user with a recreational and/or medicinal effect when inhaled. Suitable chemical and/or physiologically active volatile compounds include the group consisting of: nicotine, cocaine, caffeine, opiates and opoids, cathine and cathinone, kavalactones, mysticin, beta-carboline alkaloids, salvinorin A together with any combinations, functional equivalents to, and/or synthetic alternatives of the foregoing.

The plant material may comprise least one plant material selected from the list including *Amaranthus dubius, Arctostaphylos uva-ursi* (Bearberry), *Argemone mexicana, Amica, Artemisia vulgaris,* Yellow Tees, *Galea zacatechichi, Canavalia maritima* (Baybean), *Cecropia mexicana* (Guamura), *Cestrum noctumum, Cynoglossum virginianum* (wild comfrey), *Cytisus scoparius, Damiana, Entada rheedii, Eschscholzia califomica* (California Poppy), *Fittonia albivenis, Hippobroma longiflora, Humulus japonica* (Japanese Hops), *Humulus lupulus* (Hops), *Lactuca virosa* (Lettuce Opium), *Laggera alata, Leonotis leonurus, Leonurus cardiaca* (Motherwort), *Leonurus sibiricus* (Honeyweed), *Lobelia cardinalis, Lobelia inflata* (Indian-tobacco), *Lobelia siphilitica, Nepeta cataria* (Catnip), *Nicotiana species* (Tobacco), *Nymphaea alba* (White Lily), *Nymphaea caerulea* (Blue Lily), Opium poppy, *Passiflora incamata* (Passionflower), *Pedicularis densiflora* (Indian Warrior), *Pedicularis groenlandica* (Elephant's Head), *Salvia divinorum, Salvia dorrii* (Tobacco Sage), Salvia species (Sage), *Scutellaria galericulata, Scutellaria lateriflora, Scutellaria nana, Scutellaria* species (Skullcap), *Sida acuta* (Wireweed), *Sida rhombifolia, Silene capensis, Syzygium aromaticum* (Clove), *Tagetes lucida* (Mexican Tarragon), *Tarchonanthus camphoratus, Tumera diffusa* (Damiana), *Verbascum* (Mullein), *Zamia latifolia* (Maconha Brava) together with any combinations, functional equivalents to, and/or synthetic alternatives of the foregoing.

Preferably the substrate is a solid substrate (as opposed to an e-liquid).

Preferably, the plant material is tobacco. Any type of tobacco may be used. This includes, but is not limited to, flue-cured tobacco, burley tobacco, Maryland Tobacco, dark-air cured tobacco, oriental tobacco, dark-fired tobacco, perique tobacco and rustica tobacco. This also includes blends of the above mentioned tobaccos.Any suitable parts of the tobacco plant may be used. This includes leaves, stems, roots, bark, seeds and flowers.

The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon).

The aerosol-forming substrate may comprise reconstituted tobacco. The substrate, especially the hollow core substrate may be formed by extrusion.

Extruded tobacco can produced by forming a liquid mixture of powered tobacco and optionally a binding agent such as a gum (e.g. xanthan, guar, arabic and/or locust bean gum). The liquid mixture is heated and then extruded through a die. The extrudate is then dried e.g. freeze-dried as per the eighth aspect described below. Flavouring may be added to the liquid mixture prior to extrusion (and freeze-drying) to provide a flavoured extruded substrate e.g. a flavoured extruded hollow core substrate. The flavourant may be provided in solid or liquid form. It may include menthol, liquorice, chocolate, fruit flavour (including e.g. citrus, cherry etc.), vanilla, spice (e.g. ginger, cinnamon) and tobacco flavour. The flavourant may be evenly dispersed throughout the aerosol-forming substrate or may be provided in isolated locations and/or varying concentrations throughout the aerosol-forming substrate.

The aerosol-forming substrate (in any aspect) may comprise one or more additives selected from humectants, flavourants, fillers, aqueous/non-aqueous solvents and binders.

Humectants are provided as vapour generators - the resulting vapour helps carry the volatile active compounds and increases visible vapour. Suitable humectants include polyhydric alcohols (e.g. propylene glycol (PG), triethylene glycol, 1,2-butane diol and vegetable glycerine (VG)) and their esters (e.g. glycerol mono-, di- or tri-acetate). They may be present in the aerosol-forming substrate in an amount between 1 and 50 wt%.

The humectant content of the aerosol-forming substrate (in any aspect) may have a lower limit of at least 1 % by weight of the plant material, such as at least 2 wt %, such as at least 5 wt %, such as at least 10 wt %, such as at least 20 wt %, such as at least 30 wt %, or such as least 40 wt %. The humectant content of the aerosol-forming substrate (in any aspect) may have an upper limit of at most 50 % by weight of the plant material, such as at most 40 wt %, such as at most 30 wt %, or such as at most 20 wt %.Preferably, the humectant content is 1 to 40 wt % of the aerosol-forming substrate, such as 1 to 20 wt %

Suitable binders are known in the art and may act to bind together the components forming the aerosol-forming substrate. Binders may comprise starches and/or cellulosic binders such as methyl cellulose, ethyl cellulose, hydroxypropyl cellulose, hydroxyethyl cellulose and methyl cellulose, gums such as xanthan, guar, arabic and/or locust bean gum, organic acids and their salts such as alginic acid/ sodium alginate, agar and pectins. Preferably the binder content is 5 to 10 wt% of the aerosol-forming substrate e.g. around 6 to 8 wt%.

Suitable fillers are known in the art and may act to strengthen the aerosol-forming substrate. Fillers may comprise fibrous (non-tobacco) fillers such as cellulose fibres, lignocellulose fibres (e.g. wood fibres), jute fibres and combinations thereof. Preferably, the filler content is 5 to 10 wt% of the aerosol-forming substrate e.g. around 6 to 9 wt%.

The aerosol-forming substrate (in any aspect) may comprise an aqueous and/or non-aqueous solvent. In some embodiments, the aerosol forming substrate has a water content of between 5 and 10 wt% e.g. between 6-9 wt% such as between 7-9 wt%.

Any of the aspects described above may provide an HNB consumable having a freeze-dried plant material substrate as described below as the eighth aspect.

In an eighth aspect, there is provided a consumable for a smoking substitute system, the consumable comprising an aerosol-forming substrate formed of a moulded porous freeze-dried mixture comprising a plant material.

The provision of a consumable having an aerosol-forming substrate comprising a freeze-dried plant material may provide the consumable with a longer life span (i.e. shelf life) than a product that is not freeze-dried. Components of the freeze-dried material may then be released by passing e.g. vapour through the aerosol-forming substrate (i.e. during use of the consumable). The components of the freeze-dried material may be entrained in the airflow or vapour flow through the aerosol-forming substrate.

The aerosol-forming substrate may have a porosity of between 20% and 70% (i.e. the fraction of volume of voids with respect to total volume). For example, the aerosol-forming substrate may have a porosity of between 40% and 50%.

In a ninth aspect, there is provided a consumable for a smoking substitute system, the consumable comprising an aerosol-forming substrate formed of a moulded mixture comprising a plant material, the substrate having a porosity of between 20% and 70%.

The provision of a moulded porous mixture may allow for the passage of airflow or vapour flow through the mixture. In this way, components of the substrate may be entrained in the airflow or vapour flow as it passes through the substrate.

The term "porosity" is used herein to describe the fraction of volume of voids with respect to total volume of the substrate. The porosity may be measured by the computed tomography method (i.e. using a CT scan to determine the volume of voids and total volume), or by the gas expansion method.

In some embodiments, the porosity of the substrate may be between 40% and 50%.

The moulded mixture of the aerosol substrate may be freeze-dried. In other words, the aerosol-forming substrate may be formed of a moulded porous freeze-dried mixture comprising a plant material as described for the eighth aspect.

The freeze-dried mixture may comprise at least one volatile compound. The freeze-dried mixture may comprise nicotine.

The aerosol-forming substrate of the consumable may be an extruded aerosol-forming substrate. That is, the substrate may be formed by way of an extrusion process in which the mixture forming the substrate is moved (e.g. pushed or pulled) through a die.

The aerosol-forming substrate may alternatively be e.g. die-pressed, rolled, etc.

An airflow path may extend through the consumable of the eighth or ninth aspects between an inlet and an outlet of the consumable (e.g. a housing of the consumable which may be as described above for the fifth to seventh aspects). The inlet may be at an upstream end of the consumable and the outlet may be at a downstream end of the consumable (e.g. housing). The airflow path may pass through at least a portion of the aerosol-forming substrate. Thus, the aerosol-forming substrate may be disposed between the inlet and the outlet.

The aerosol-forming substrate of the eighth/ninth aspect may comprise upper and lower surfaces spaced by opposing longitudinally-extending transverse surfaces wherein the depth of the substrate (between the upper and lower surfaces) and the width of the substrate (between the opposing transverse surfaces) are unequal e.g. the width is greater than the depth. Thus the substrate may be as described in the first to third aspects

In other embodiments of the eighth and ninth aspects, the substrate may be cylindrical (i.e. rod-shaped).

In a tenth aspect, there is provided a method of forming an aerosol-forming substrate for a smoking substitute system, the method comprising: forming a mixture including plant material, a volatile compound, and water; moulding the mixture to form a desired shape; and freeze-drying the moulded mixture.

Moulding the mixture may comprise extruding the mixture (i.e. forcing the mixture through a die) and/or die-pressing the mixture. The moulding may be performed so as to form a moulded mixture having a circular or rectangular cross-sectional shape.

Where the moulding comprises extrusion, the moulding may form an elongate moulded body. The method may thus further comprise a cutting process for cutting the elongate moulded body into a plurality of moulded mixture portions. The cutting process may be performed prior to or after the freeze drying.

Freeze drying the mixture may be performed so as to substantially remove all moisture from the moulded mixture. The freeze drying process may be performed so as to result in a solid (e.g. self-supporting) body. The freeze drying process may be performed as to produce a porous body. That is the removal of moisture from the moulded moisture may form gaps in the moulded mixture. The freeze drying may be performed so as to provide a freeze-dried moulded mixture having a porosity of between 20% and 70% (or between 40% and 50%).

The volatile compound of the mixture may be nicotine. The mixture may comprise a plurality of volatile compounds. For example, the mixture may comprise one or more of the volatile compounds listed above with respect to the first aspect.

The plant material may be tobacco. The mixture may comprise a plurality of plant materials. For example, the mixture may comprise one or more of the plant materials (or tobaccos) listed above with respect to the first aspect.

The mixture may comprise one or more additives selected from humectants, flavourants, fillers, solvents and binders. These may be as described above with respect to the earlier aspects.

In an eleventh aspect, there is provided a smoking substitute system comprising:
a consumable as described above with respect to the eighth or ninth aspect; and
a vapour generating article upstream of the consumable and in fluid communication with the substrate of the consumable

The vapour generating article may comprise a passage for fluid flow therethrough. The passage may extend through (at least a portion of) the vapour generating article, between openings that may define an inlet and an outlet of the passage. The passage may be fluidly connected to the airflow path of the consumable. In this respect, the outlet of the passage may be in fluid communication (and may be adjacent to) the substrate of the consumable. In this respect, a user may draw fluid (e.g. air) into and through the passage by inhaling at the outlet of the consumable (i.e. using the mouthpiece). The air may pass from the passage and through the substrate to the outlet.

The vapour generating article may comprise a tank (reservoir) for containing a vaporisable liquid (e.g. an e-liquid). The e-liquid may, for example, comprise a base liquid and e.g. nicotine. The base liquid may include propylene glycol and/or vegetable glycerine.

The e-liquid may further comprise a flavourant. The flavourant may be natural or synthetic. For example, the flavourant may include menthol, liquorice, chocolate, fruit flavour (including e.g. citrus, cherry etc.), vanilla, spice (e.g. ginger, cinnamon) and tobacco flavour. The flavourant may be evenly dispersed or may be provided in isolated locations and/or varying concentrations.

The tank may be defined by a tank housing. At least a portion of the tank housing may be translucent. For example, the tank housing may comprise a window to allow a user to visually assess the quantity of e-liquid in the tank. The tank may be referred to as a "clearomizer" if it includes a window, or a "cartomizer" if it does not. A passage may extend longitudinally within the tank and a passage wall may define the inner wall of the tank. In this respect, the tank may surround the passage e.g. the tank may be annular. The passage wall may comprise longitudinal ribs extending therealong. These ribs may provide support to the passage wall. The ribs may extend for the full length of the passage wall. The ribs may project (e.g. radially outwardly) into the tank.

The vapour generating article may comprise a vaporiser. The vaporiser may comprise a wick. The vaporiser may further comprise a heater. The wick may comprise a porous material. A portion of the wick may be exposed to fluid flow in the passage. The wick may also comprise one or more portions in contact with liquid stored in the reservoir. For example, opposing ends of the wick may protrude into the reservoir and a central portion (between the ends) may extend across the passage so as to be exposed to fluid flow in the passage. Thus, fluid may be drawn (e.g. by capillary action) along the wick, from the reservoir to the exposed portion of the wick.

The heater may comprise a heating element, which may be in the form of a filament wound about the wick (e.g. the filament may extend helically about the wick). The filament may be wound about the exposed portion of the wick. The heating element may be electrically connected (or connectable) to a power source. Thus, in operation, the power source may supply electricity to (i.e. apply a voltage across) the heating element so as to heat the heating element. This may cause liquid stored in the wick (i.e. drawn from the tank) to be heated so as to form a vapour and become entrained in fluid flowing through the passage. In some cases, this vapour may subsequently cool to form an aerosol in the passage.

This vapour (or aerosol) may then pass through the substrate (i.e. downstream of the vapour generating article). Upon passing through the substrate, the vapour may at least partially rehydrate the freeze-dried material such that one or more components (e.g. nicotine) of the substrate become entrained in the vapour. The combined vapour/aerosol may then pass out of the consumable via the outlet so as to be inhaled by a user.

In some embodiments, the consumable and the vapour generating article may be enclosed in a shared housing (e.g. such as the housing described above with respect to the first aspect). In such an embodiment, the vapour generating article may be disposed at an upstream end of the housing and an airflow path may be defined through the vapour generating article and the consumable to an outlet of the housing. In this respect, the vapour generating article may form part of the consumable.

Alternatively, in other embodiments the vapour generating may be separate form, but engageable with, the vapour generating article (e.g. by way of an interference fit, snap-engagement, etc.). In this respect, the consumable and vapour generating articles may be interchangeable with other consumables/vapour generating articles.

The system may further comprise a main body. The main body and the vapour generating article may be configured to be physically coupled together. For example, the vapour generating article may be at least partially received in a recess of the main body, such that there is snap engagement between the main body and the vapour generating article. Alternatively, the main body and the vapour generating article may be physically coupled together by screwing one onto the other, or through a bayonet fitting.

Thus, the vapour generating article and/or consumable may comprise one or more engagement portions for engaging with a main body. In this way, one end of the vapour generating article (i.e. the inlet end) may be coupled with the main body, whilst an opposing end (i.e. the outlet end) of the vapour generating article may define a mouthpiece.

The main body and the vapour generating article and/or consumable may be configured to be physically coupled together. For example, the vapour generating article may be at least partially received in a recess of the main body, such that there is snap engagement between the main body and the vapour generating article. Alternatively, the main body and the vapour generating article may be physically coupled together by screwing one onto the other, or through a bayonet fitting.

Thus, the vapour generating article may comprise one or more engagement portions for engaging with a main body. In this way, one end of the vapour generating article (i.e. the inlet end) may be coupled with the main body, whilst an opposing end of the vapour generating article may be coupled with the consumable.

The main body, consumable or the vapour generating article may comprise a power source or be connectable to a power source. The power source may be electrically connected (or connectable) to the heater. The power source may be a battery (e.g. a rechargeable battery). An external electrical connector in the form of e.g. a USB port may be provided for recharging this battery.

The vapour generating article may comprise an electrical interface for interfacing with a corresponding electrical interface of the main body. One or both of the electrical interfaces may include one or more electrical contacts. Thus, when the main body is engaged with the vapour generating article, the electrical interface may be configured to transfer electrical power from the power source to a heater of the vapour generating article. The electrical interface may also be used to identify the vapour generating article from a list of known types. The electrical interface may additionally or alternatively be used to identify when the vapour generating article and/or the consumable is connected (e.g. directly or indirectly) to the main body.

The main body may alternatively or additionally be able to detect information about the consumable via an RFID reader, a barcode or QR code reader. This interface may be able to identify a characteristic (e.g. a type) of the consumable. In this respect, the consumable may include any one or more of an RFID chip, a barcode or QR code, or memory within which is an identifier and which can be interrogated via the interface.

The vapour generating article, consumable or main body may comprise a controller, which may include a microprocessor. The controller may be configured to control the supply of power from the power source to the heater (e.g. via the electrical contacts). A memory may be provided and may be operatively connected to the controller. The memory may include non-volatile memory. The memory may include instructions which, when implemented, cause the controller to perform certain tasks or steps of a method.

The vapour generating article, consumable or main body may comprise a wireless interface, which may be configured to communicate wirelessly with another device, for example a mobile device, e.g. via Bluetooth^{®}. To this end, the wireless interface could include a Bluetooth^{®} antenna. Other wireless communication interfaces, e.g. WiFi^{®}, are also possible. The wireless interface may also be configured to communicate wirelessly with a remote server.

An airflow (i.e. puff) sensor may be provided that is configured to detect a puff (i.e. inhalation from a user). The airflow sensor may be operatively connected to the controller so as to be able to provide a signal to the controller that is indicative of a puff state (i.e. puffing or not puffing). The airflow sensor may, for example, be in the form of a pressure sensor or an acoustic sensor. The controller may control power supply to the heater in response to airflow detection by the sensor. The control may be in the form of activation of the heater in response to a detected airflow. The airflow sensor may form part of the consumable or the main body.

In an alternative embodiment the vapour generating article may be integral with the main body. In such embodiments, an aerosol former (e.g. e-liquid) of the vapour generating article may be replenished by re-filling the tank of the vapour generating article (rather than replacing the vapour generating article). Access to the tank (for re-filling of the e-liquid) may be provided via e.g. an opening to the tank that is sealable with a closure (e.g. a cap).

The vapour generating article may be in the form of an e-cigarette consumable (e.g. pod). The main body may be an e-cigarette device.

In a twelfth aspect, there is provided a method of using a substitute smoking system according to the eleventh aspect, the method comprising: engaging the consumable with the vapour generating article; generating a vapour using the vapour generating article; and causing the vapour to flow through the aerosol-forming substrate of the consumable.

Once consumed the consumable may be released from the vapour generating article and a further consumable may subsequently be (releasably) engaged with the vapour generating article for receipt of vapour.

The consumable described above for any of the first to seventh aspects may be coupled with a heating element in a heat not burn (HNB) device.

Accordingly in a thirteenth aspect, there is provided a heat not burn (HNB) system comprising:
a heat not burn consumable as described above any of the first to seventh aspects; and a device comprising at least one heating element.

The device may be a HNB device i.e. a device adapted to heat but not combust the aerosol-forming substrate. The device may comprise a device housing for housing the heating element(s). The heating element(s) may comprise an elongated e.g. rod, tube-shaped or blade heating element. The heating element(s) may project into or surround a cavity within the device housing for receiving the consumable described above.

When the upstream end wall is a pierceable membrane, the heating element may be configured to pierce the pierceable membrane when engaged with the consumable. The at least one aperture of the pierceable membrane (of the consumable) may be located such that it is spaced from the heating element when the heating element is received in the substrate. That is, the at least one aperture may be positioned at a region of the pierceable membrane that is not pierced by the heating element when inserted into the substrate for heating of the substrate. For example, the heating element may be arranged so as to pierce a central region (or e.g. the centre) of the pierceable membrane. In this way, the at least one aperture may permit airflow through the pierceable membrane when the heating element is received therethrough.

The device may further comprise a PCB connected to the heating element(s) for controlling the temperature of the heating element(s). It may further comprise a battery e.g. a recyclable battery such as a 2000mAh battery.

In some embodiments, the device comprises a first heating element for facing/abutting/overlying the upper or lower surface of the substrate. The device may comprise a second heating element which, when the consumable is engaged, faces/abuts/overlies the other of the upper and lower surface of the substrate. In some embodiments, the device comprises a core heating element for penetrating the substrate or for being received in the hollow core recess of the substrate.

The at least one heating element (e.g. first/second/core heating element) may be a planar heating element. It may have a greater width and length than depth. The length and width may be equal but, preferably, the length is greater than the width such that the planar heating element is a rectangular element i.e. has a substantially rectangular upper and lower planar surfaces. The length of the planar heating element may be between 10 and 20 mm e.g. between 10 and 15 mm. The width of the planar heating element may be between 7 and 14 mm e.g. between 7 and 12 mm or 7 and 10 mm e.g. around 8 mm. The depth of the planar heating element may be between 0.5 and 2 mm, e.g. around 1 mm.

The first/second/core heating element may be a ceramic heating element.

The heat not burn (HNB) device may comprise: a device housing; and at least one heating element, the at least one heating element being housed within a cavity at a first longitudinal end of the device housing, the device housing have a first longitudinal end face defining an aperture in communication with said cavity,
wherein the device further comprises a sealing plate movable from a first position in which the aperture is open to a second position in which the aperture is at least partially sealed by the sealing plate.

The sealing plate may be slidable (e.g. slidable in an axial direction) from the first position to the second position.

In the first position, the sealing plate forms a base of the cavity with the at least one heating element extending towards the aperture through the sealing plate. The sealing plate may be an apertured plate, so that as the sealing plate moves from the first to the second position, the at least one heating element passes through the aperture.

The device housing may comprise at least one channel and the sealing plate may comprise at least one transverse tab extending from the sealing plate through the channel to rest on an exterior of the device housing. The device housing may comprise two opposing channels and the sealing plate may comprise two opposing transverse tabs. The transverse tab(s) may be used to manually move the sealing plate between the first and second positions.

The device housing (and the cavity) may have a substantially rectangular or obround transverse cross-section.

The device is adapted to receive a consumable (which is as described above) and which is insertable into the device housing for engagement with the at least one heating element (which may be a first/second/core heating element as described above). Where the consumable comprises a housing, the consumable is inserted with the second longitudinal end wall of the housing protruding from the device housing.

The consumable is inserted when the sealing plate is in its first position. After use, the sealing plate is moved to its second position which forces the consumable from the chamber and, ultimately blocks the aperture at the first longitudinal end face of the device housing so that the user is prevent from contacting the hot heating element.

In a fourteenth aspect, there is provided a method of using a heat not burn system according to the thirteenth aspect, the method comprising: inserting the consumable into the device; and heating the consumable using the heating element.

In some embodiments, the method comprises inserting the consumable into a cavity within the device housing and penetrating the consumable with a core heating element upon insertion of the consumable. For example, the core heating element (e.g. the planar core heating element) may penetrate the aerosol-forming substrate in the consumable e.g. by being received within the hollow core recess/planar recess of the substrate. The core heating element may be received in the housing through the upstream longitudinal end face of the housing. Where there is an upstream longitudinal end wall, the core heating element may be received in the housing through the aperture. Where there is a membrane/foil sealing the upstream longitudinal end face of the housing or the aperture, the membrane is removed or pierced to allow insertion of the core heating element into the housing. In some embodiments, when the consumable comprises an end wall in the form of a pierceable membrane, the heating element pierces the pierceable membrane of the consumable when the consumable is inserted into the device.

The core heating element (e.g. the planar core heating element) may penetrate the aerosol-forming substrate in the consumable e.g. by being received within the hollow core recess/planar recess of the substrate.

The insertion of the consumable may be performed such that the at least one aperture in the end wall (e.g. pierceable membrane) is spaced from the heating element when the heating element is received through the end wall.

The core heating element may be received in the housing through the upstream longitudinal end face of the housing. Where there is an upstream longitudinal end wall, the core heating element may be received in the housing through the aperture.

In other embodiments, the method comprises inserting the consumable into the cavity within the device housing so that the first heating element overlies the upper surface of the substrate e.g. in abutment with the upper wall of the housing. The method may further comprise inserting the consumable into the cavity so that the second heating element overlies the lower surface of the substrate e.g. in abutment with the lower wall of the housing.

Once consumed the consumable may be released from the or each heating element and a further consumable may subsequently be (releasably) engaged with the or each heating element of the device for heating.

The disclosure includes the combination of the aspects and preferred features described except where such a combination is clearly impermissible or expressly avoided.

### Summary of the figures

So that the disclosure may be more readily understood, and so that further features thereof may be appreciated, embodiments and experiments illustrating the principles of the disclosure will now be described by way of example with reference to the accompanying figures in which:
Figure 1 shows a first embodiment of a consumable comprising a planar slab of plant product;
Figure 2 shows cuboid brick of plant product;
Figures 3a and 3b show a housing for a consumable;
Figure 4a and 4b shows a second embodiment of a consumable comprising a planar slab of plant product;
Figures 5a - 5c show a third embodiment of a consumable;
Figures 6a and 6b show a fourth embodiment of a consumable;
Figure 7 shows the fourth embodiment with a core heating element inserted;
Figure 8 shows a lateral cross section through the third embodiment with a core heating element inserted;
Figures 9-11 show a device according to an embodiment;
Figure 12 shows a fifth embodiment of a consumable;
Figure 13 shows a longitudinal cross section through a sixth embodiment of a consumable;
Figure 14 shows a longitudinal cross section through a seventh embodiment of a consumable;
Figure 15 shows a perspective internal view of an eighth embodiment of a consumable; and
Figure 16 shows the downstream longitudinal end wall of the housing of the embodiment shown in Figure 15;
Figures 17a - 17g show alternative transverse cross sections of an aerosol-forming substrate or housing or filter;
Figure 18 shows a ninth embodiment of a consumable;
Figure 19 shows an expanded view of a housing of a consumable;
Figure 20 shows a part of the housing of Figure 19;
Figures 21a and 21b illustrate a method of manufacturing the housing of Figure 19;
Figure 22 shows an expanded view of a housing of a consumable;
Figure 23 shows a part of the housing of Figure 22; and
Figures 24a and 24b are schematic views of a smoking substitute system.

### Detailed Description

Aspects and embodiments of the disclosure will now be discussed with reference to the accompanying figures. Further aspects and embodiments will be apparent to those skilled in the art.

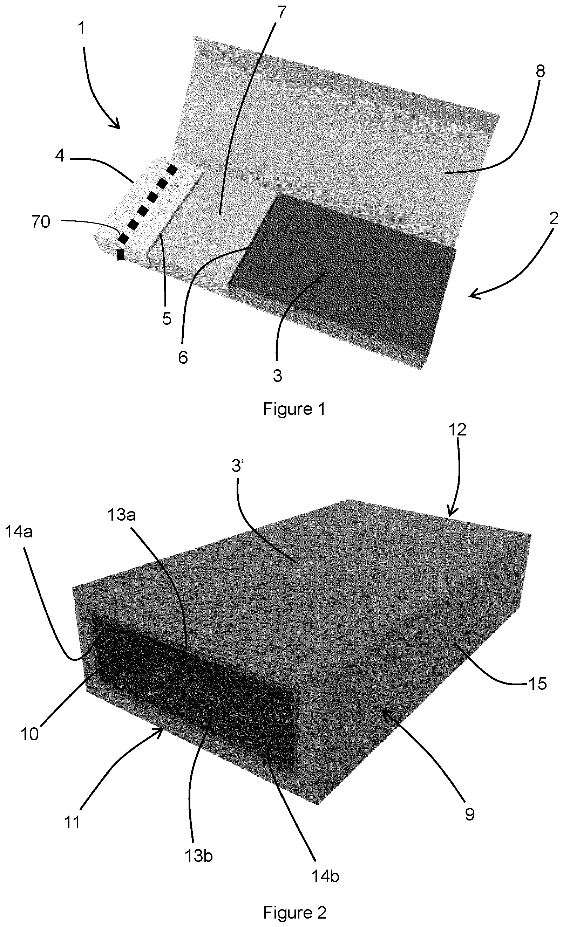

Figure 1 shows a perspective view of a first embodiment of a heat not burn (HNB) consumable 1 comprising a planar substrate 2 of reconstituted tobacco such that the consumable 1 has a substantially rectangular transverse cross section.

By providing the reconstituted tobacco as a planar substrate (having a substantially rectangular transverse cross section) rather than as a cylindrical rod (having a substantially circular cross section), the reconstituted tobacco has a greater exposed surface area for contact with a heating element thus allowing quicker and more even heat transfer from the heating element to the reconstituted tobacco. In this manner, heating of the reconstituted tobacco can be effected using a heating element at a lower temperature (e.g. around 250 °C) which reduces the chances of burning of the reconstituted tobacco.

The planar substrate 2 has a substantially rectangular upper surface 3, and has a length of around 12 mm, a width of around 8 mm and a depth of around 6 mm.

The consumable further comprises a filter 4. The filter 4 is also formed as a planar slab having a substantially rectangular transverse cross section. The filter 4 typically has a width of around 8 mm, a depth of around 6 mm and a length of around 3 mm.

The filter 4 has an upstream longitudinal end face 5 which faces (but is spaced from) the downstream longitudinal end face 6 of the planar slab 2 of reconstituted tobacco. The filter 4 is provided with a plurality of axially aligned openings 70 which each form part of a respective inwardly-directed air flow path extending into the filter 4.

The consumable 1 further comprises a paper spacer 7 interposed between the filter 4 and the plant product 2. The spacer 7 typically has a width of around 8 mm, a depth of around 6 mm and a length of around 5 mm.

The consumable 1 further comprises a paper wrapper 8 which is shown open in Figure 1 but which is wrapped around to fully enclose the upper surface 3, lower surface and the opposing transverse surfaces of the consumable 1.

This consumable 1 of Figure 1 may be heated using a first heating element in abutment with the upper surface 3 and/or a second heating element in abutment with the lower surface. The planar slab 2 is then heated and externally and inwards from the upper and/or lower surfaces.

Figure 2 shows a portion of reconstituted tobacco extruded into a cuboid brick 9. The cuboid brick has an upper surface 3' and a lower surface (not visible) spaced by opposing transverse surfaces 15 (only one visible). The outer surfaces of the cuboid brick 9 are coated with a hydrophobic/liquid impermeable coating.

The length of the cuboid brick is typically around 12 mm, with a depth of around 6 mm and width of around 10 mm.

The cuboid brick 9 has a hollow core defined by a cuboid recess 10 extending in a length direction from the upstream longitudinal end face 11 of the cuboid brick 9 to the opposing downstream longitudinal end face 12.

The cuboid recess 10 is defined by upper and lower inner surfaces 13a, 13b and opposing inner transverse surfaces 14a, 14b. The cuboid recess has a depth of around 1 mm, a width of around 8 mm and a length of around 12 mm.

The reconstituted tobacco at or proximal one or more of the upper/lower/transverse inner surfaces 13a, 13b, 14a, 14b defining the cuboid recess 10 may be dosed with an e-liquid which may contain aerosol formers such as polyglycol (PG) and/or vegetable glycerine (VG).

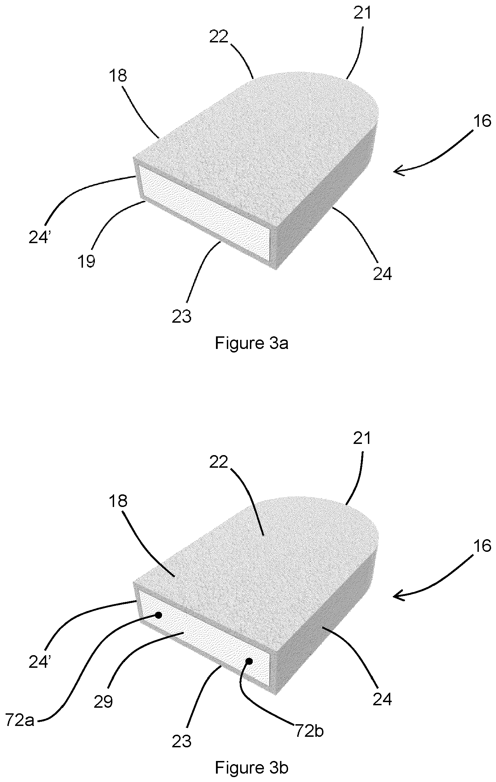

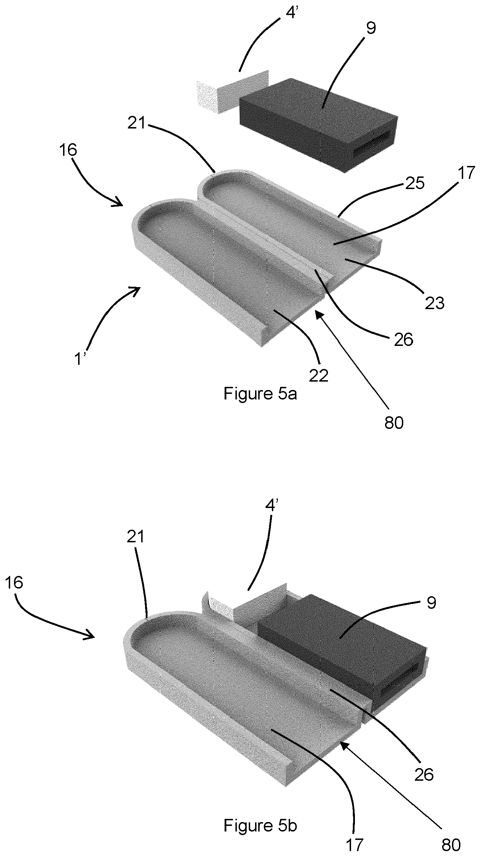

As can be seen in Figures 5a-5c, the cuboid brick 9 can be inserted and glued into a substantially rigid, self-supporting housing 16 having walls of a uniform thickness of around 2 mm formed of moulded bagasse pulp to form the consumable 1'. The housing 16 has a textured e.g. meshed inner surface 17 facing the reconstituted tobacco. The outer surface 18 (visible in Figures 3a and 3b) of the housing 16 is substantially smooth.

As also seen in Figures 3a/3b, the housing 16 is a hollow cuboid housing defining a cuboid chamber 20 which is dimensioned to receive the cuboid brick 9 of reconstituted tobacco.

As seen in Figures 3a and 3b, the housing 16 has an open upstream longitudinal end face 19 having a rectangular transverse cross-section. It has a rounded opposing downstream longitudinal end wall 21 which has at least one mouthpiece aperture (not visible).

The housing 16 has an upstream longitudinal end having an opening 80 (see e.g. Figure 5a-5b) with a rectangular transverse cross-section. As is also apparent from Figures 3a/3b, the opening 80 is substantially sealed by an upstream longitudinal end wall in the form of a pierceable membrane 29 that extends across the opening 70. This pierceable membrane 29 retains the substrate 2 within the housing 16 prior to use.

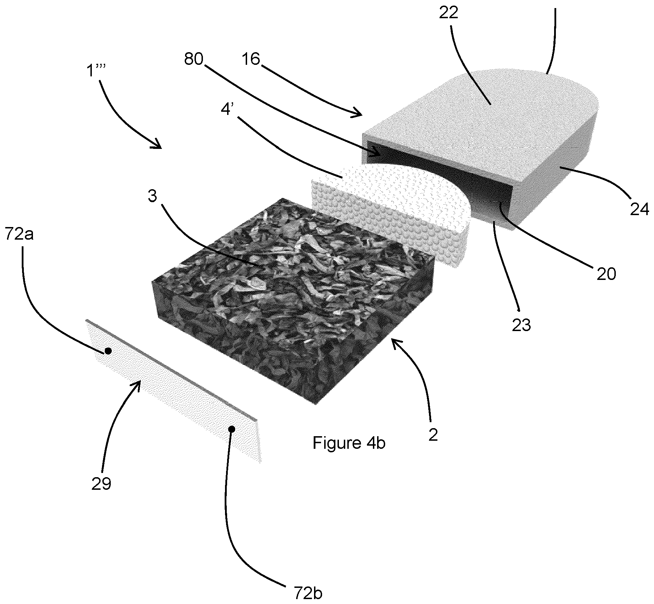

In the embodiment shown in Figures 3b/4b, the pierceable membrane 29 comprises two apertures 72a, 72b that are spaced laterally from one another so as to be located either side of a centre of the pierceable membrane 29. In particular, the apertures 72a, 72b are located such that when a heating element (discussed further below) is received therethrough (and into the substrate 2), the apertures 72a, 72b are spaced from the heating element. In this way, the apertures 72a, 72b permit airflow through the substrate 2, even when the heating element is received therein.

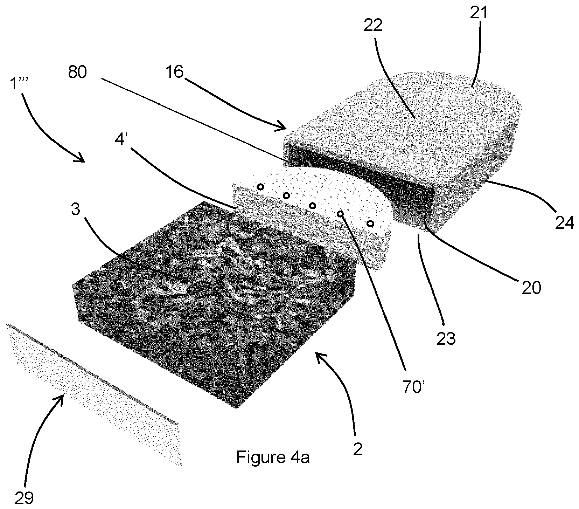

The consumable 1' has a filter 4' having a rounded downstream longitudinal end face for abutment with the inside surface of the downstream longitudinal end wall 21 of the housing 16. In the embodiment shown in Figure 4a, the filter 4' has a plurality of inwardly directed air flow paths having openings 70' on its upper surface.

The downstream longitudinal end wall 21 at least partly (and preferably completely) obscures the filter 4' from view by a user.

By concealing the filter 4' from view, the user is not exposed to the residues remaining in the filter 4'/consumable 1' after use thus improving the aesthetic appeal of the consumable after use and avoiding transfer of residue to the user.

The housing 16 comprises an upper wall 22 and lower wall 23 spaced by opposing transverse walls 24. One of the opposing transverse walls 24 comprises a longitudinally extending junction 25 and the downstream longitudinal end wall 21 also comprises a junction such that the housing can be opened to expose the cuboid chamber as shown in figures 5a-5c.

The other opposing transverse wall 24' comprises a longitudinally extending hinge portion 26 such that the housing 16 may be opened along the junctions by pivoting of the upper wall 22 and lower wall 23 about the hinge portion 26.

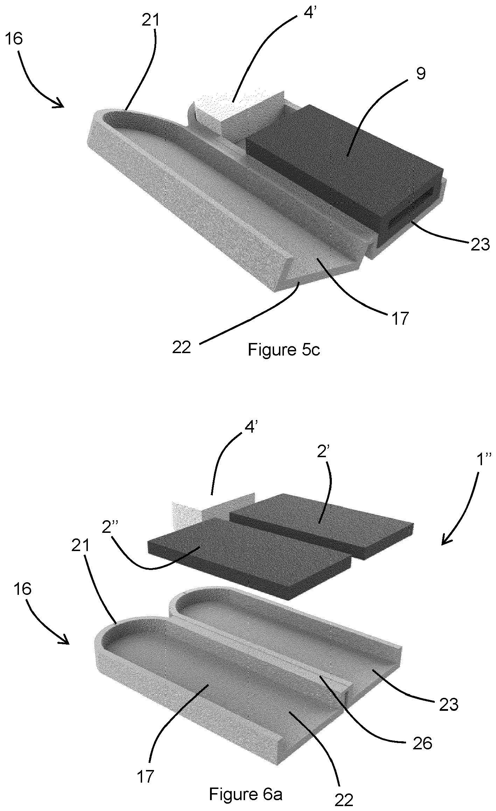

In this way, the cuboid brick 9 and the filter 4' can be fitted (and glued) into the opened housing 16 and then the housing closed (by pivoting about the hinge portion 26 as shown in Figure 5c). The junctions can be sealed e.g. with glue.

The opening 80 is then sealed with the pierceable seal 29 (i.e. by adhering the pierceable seal 29 to the upper 22, lower 23 and transverse 24, 24' walls). In this way, the closed housing 16 has the shape shown in Figures 3a/3b and, with such a housing 16, the consumable 1' becomes more akin to a cartridge or "pod" that effectively contains residue after use to protect a user from contamination.

Furthermore, the housing 16 is provided on its outer surface i.e. on the outer surface of the upper wall 22, lower wall 23, opposing transverse walls 24 and downstream longitudinal end wall 21 with a hydrophobic surface or coating. This helps prevent transfer of the e-liquid from the substrate (i.e. cuboid brick 9) to the user. It also helps prevent tackiness around the mouthpiece aperture in the downstream longitudinal end wall 21.

Figures 6a-6b shown another embodiment in which the consumable 1" comprises two planar substrates 2', 2" of reconstituted tobacco, each having a depth of around 2 mm, a length of around 12 mm and a width of around 10 mm.

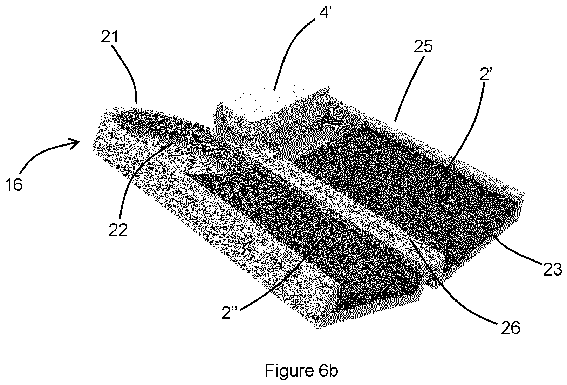

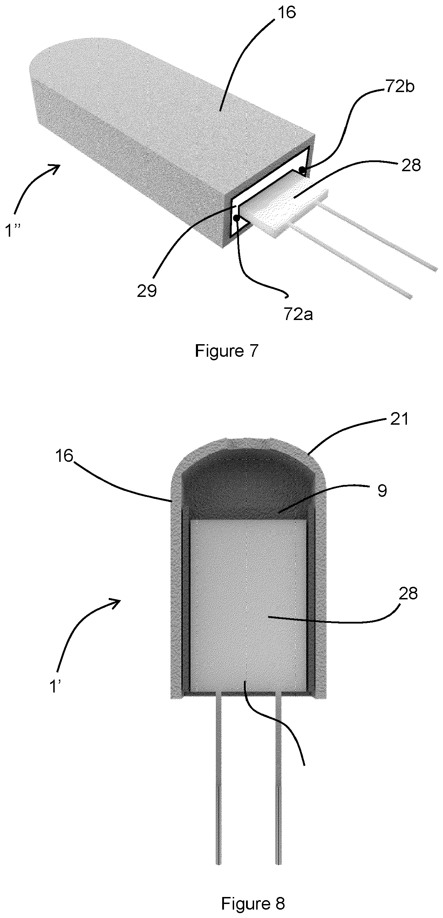

The two planar substrates 2', 2" are vertically and horizontally aligned and spaced from one another to define a planar recess 27 (visible in Figure 7). The housing 16 is as described previously and the two planar substrates 2', 2" are each glued into a respective half of the housing 16 (see Figure 6b) before closing the housing 16 by pivoting along the hinge portion 26 to form the planar recess 27. The tobacco at or proximal the inner surface (facing the planar recess 27) on one or both of the planar slabs 2', 2" is dosed with e-liquid as described above. The inner surface of the housing 16 is coated with a hydrophobic coating or textured to provide a hydrophobic surface to prevent seepage of the e-liquid from the substrates 2', 2" through the housing. The junctions around the housing 16 are sealed using glue.

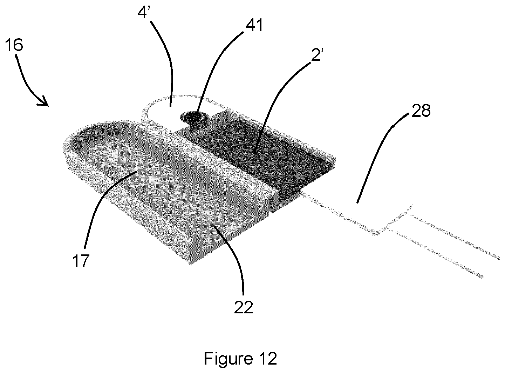

Figure 12 shows a variation (one planar substrate omitted from view) where the filter 4' (shown in cross-section) comprises a liquid release member 41 containing an aerosol former such as vegetable glycerine and/or propylene glycol at its first longitudinal end face. The release member 41 is configured to release the aerosol former during use (e.g. by providing an envelope that is breakable upon abutment with the heating element 28 or that melts during heating) so that upon release, the aerosol former can penetrate the plant product.

The closed housing 16 is seen in Figure 7 with a planar, ceramic heating element 28 inserted into the planar recess 27. As is apparent from Figure 7, the apertures 72a, 72b of the pierceable membrane 29 are laterally spaced from the heating element 28 when the heating element is inserted into the recess (not visible in the figure).

Figure 8 shows a longitudinal cross section of the housing 16 containing the cuboid brick 9 of reconstituted tobacco.

As can be seen, when inserted into the recess 27, the heating element 28 can contact the surfaces of the cuboid recess 10 or planar recess 27 thus allowing quicker heating. It can also be seen that, in all embodiments, the depth of the reconstituted tobacco between the heating surface(s) and the opposing surfaces is substantially constant in the depth direction which results in a more even heat transfer from the heating element 28 to the reconstituted tobacco. In this manner, heating of the tobacco can be effected using a heating element 28 at a lower temperature (e.g. around 250 °C) which reduces the chances of burning of the plant product.

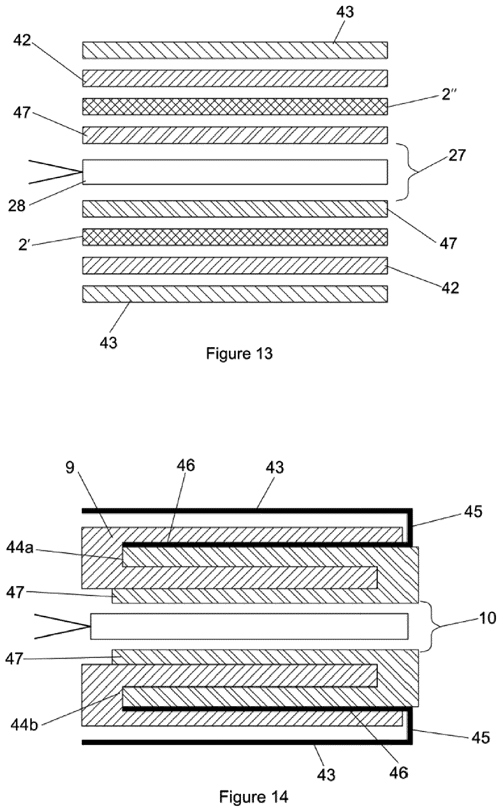

Figures 13 and 14 show further embodiments where the planar recess 27 or cuboid recess 10 is fully lined with a thermally conductive material such as aluminium foil 47. The aluminium foil 47 fully lines the recess 10, 27 and overlies the plant product which may be in the form of two planar substrates 2', 2" (as shown in Figure 13) or may be a cuboid brick 9 (as shown in Figure 14).

In the embodiment shown in Figure 13, the opposing outer surfaces of the planar substrates 2', 2" are further lined with a dimpled foil 42 (which is liquid impermeable). The plant product and foil layers 47, 42 are enclosed with cardboard wrapper 43 (although it may also be formed of moulded pulp, e.g. moulded bagasse pulp).

The heating element 28 is received within the planar recess 27 and the aluminium foil 47 increases heat transfer to the plant product. The dimpled foil 42 forms a liquid impermeable barrier to prevent seepage of any e-liquid dosed into the plant product into the cardboard wrapper, the dimples acting to increase air flow through the consumable upon inhalation by the user.

In Figure 14, the aluminium foil 47 fully lines the cuboid recess 10 and then extends over the downstream longitudinal end face 12 of the cuboid brick and axially into channels 44a, 44b formed within the downstream longitudinal end face 12. Thus the aluminium foil 47 partly covers the downstream longitudinal end face 12 of the cuboid brick.

The cuboid brick 9 and foil layers 47, 42 are enclosed with a cardboard wrapper 43 (although it may also be formed of moulded pulp, e.g. moulded bagasse pulp). The wrapper 43 comprises a transverse extension 45 which extends to cover a portion of the downstream longitudinal end face 19 of the cuboid brick 9. The transverse extension 45 comprises an inwardly-depending axial extension 46 extending inwards into the channels 44a, 44b in the plant product in abutment with the aluminium foil 47.

The heating element 28 is received within the cuboid recess 10 and the aluminium foil 47 increases heat transfer to the plant product.

A further embodiment of a consumable 1‴ is shown in Figure 4 where the housing 16 and filter 4' are as previously described. The reconstituted tobacco is formed as single planar substrate 2 having a substantially rectangular upper surface 3. The substrate 2 has a length of around 12 mm, a width of around 8 mm and a depth of around 6 mm.

The upstream longitudinal end of the housing 16 is again provided with an end wall in the form of a pierceable membrane such as a metallic foil or plastic membrane 29. The membrane 29 comprises apertures 72a, 72b that are centrally located between the upper 22 and lower 23 walls and that are laterally spaced either side of the centre of the membrane 29. The membrane obscures the reconstituted tobacco from view by a user prior to use and retains the tobacco within the housing 16. When the consumable 1‴ is used, the membrane is pierced by the insertion of the heating element 28. When inserted, the apertures 72a, 72b are laterally spaced from the heating element 28 so as to permit airflow through the substrate. The apertures 72a, 72b are sized and shaped so as to retain the substrate 2 within the housing 16, but allow the passage of air through the pierceable membrane 29.

For this embodiment, the heating element could be a heating blade that pierces the planar substrate 2.

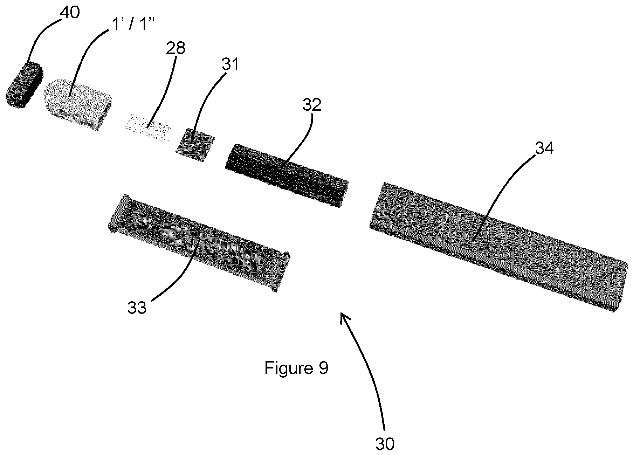

Figure 9 shows a heat not burn (HNB) device 30 comprising the heating element 28 which is mounted on and controlled by a PCB 31 connected to a battery 32, the PCB 31 and battery 32 being housed within an electrical sleeve 33. In turn electrical sleeve 33 and heating element 28 are housed within (and fully enclosed by) a device housing 34. The device housing 34 has a chamber 35 at its first longitudinal end which has an aperture at its first longitudinal end face and which houses the heating element 28.

The consumable 1'/1"/1‴ is insertable into the chamber 35 within the device housing 34 such that the heating element 28 is received in the housing 16 (e.g. within the planar recess 26 or cuboid recess 10 within the reconstituted tobacco) via the upstream longitudinal end face 19 of the housing 16. The downstream longitudinal end wall 21 of the housing 16 protrudes from the device housing 34.

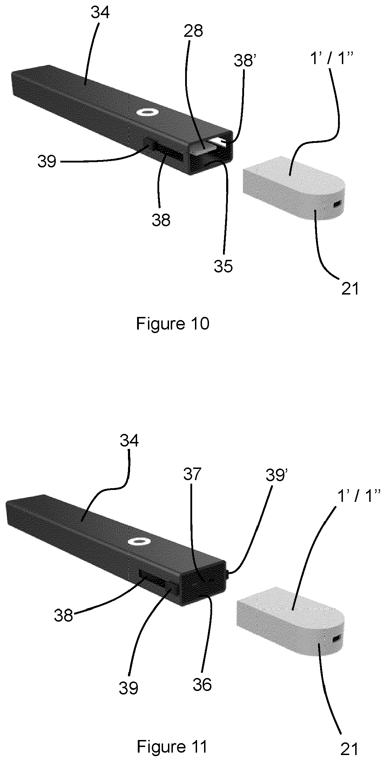

The device 30 further comprises a sealing plate 36 movable (slidable in a axial direction) from a first position (shown in figure 10) in which the aperture at the upstream longitudinal end face of the device housing 34 is open, to a second position (shown in Figure 11) in which the aperture is at least partially sealed by the sealing plate 36.

In the first position, the sealing plate 36 forms a base of the chamber 35 with the heating element 28 extending towards the aperture through the sealing plate 36. The sealing plate 36 has a slit 37, so that as it moves from the first to the second position, the heating element 28 passes through the slit.

The device housing 34 has two opposing channels 38, 38' and the sealing plate 36 comprises two opposing transverse tabs 39, 39' extending from the sealing plate 36 through the channels 38, 38' to rest on an exterior of the device housing 34. The transverse tabs 39, 39' may be used to manually move the sealing plate 36 between the first and second positions.

The consumable 1'/1"/1‴ is inserted when the sealing plate 36 is in its first position. The heating element 28 lies within the cuboid recess 10 or the planar recess 27 and the user activates the heating element e.g. by an actuator button located on the device housing 34. The device housing 34 may also comprise an indicator showing when the heating element 28 had reached the correct temperature (250°C).

The user then places the downstream longitudinal end wall 21 of the consumable 1'/1"/1‴ into their mouth and draws on the consumable 1'/1"/1‴ in order to inhale an aerosol containing nicotine.

After use, the sealing plate 36 is moved to its second position which forces the consumable 1'/1"/1‴ from the chamber 35 and ultimately blocks the aperture at the upstream longitudinal end face of the device housing 34 so that the user is prevent from contacting the hot heating element 28.

The device 30 may further comprise a cap 40 e.g. a magnetic cap for sealing the aperture at the upstream longitudinal end face of the device housing e.g. when the device is not in use for an extended period. The cap 40 may have a recess on its underside such that the aperture can be sealed with a consumable 1'/1"/1‴ in situ.

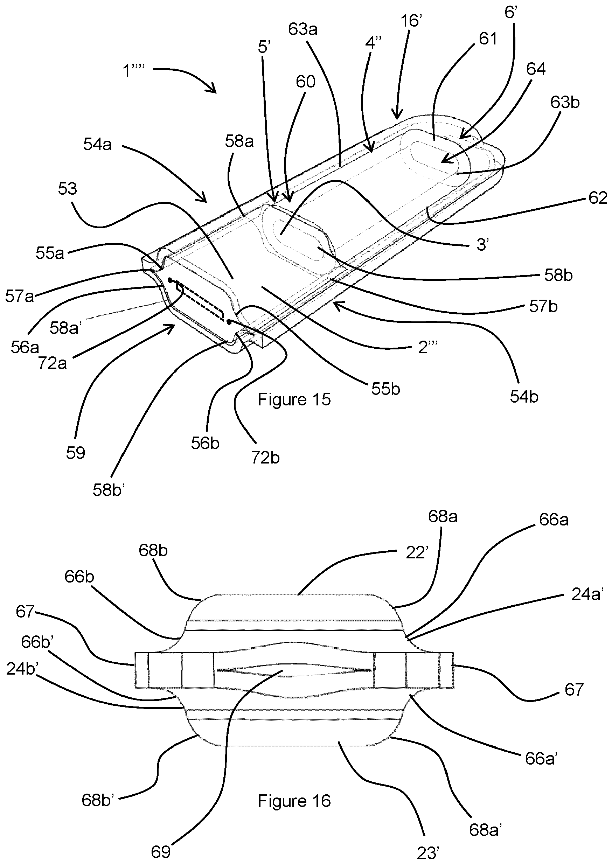

Figure 15 shows a perspective internal view of an eighth embodiment of a consumable 1ʺʺ. This embodiment may also be used with the device 30 as described above.

The planar substrate 2‴ comprises a planar upper surface 3' and a planar lower surface 53 spaced by opposing longitudinally-extending transverse surfaces 54a, 54b. The depth of the substrate 2‴ (between the upper and lower surfaces, 3', 53) and the width of the substrate 2‴ (between the opposing transverse surfaces 54a, 54b) are unequal with the width being greater than the depth i.e. the aspect ratio of the width to depth is greater than 1:1.

The opposing transverse surfaces 54a, 54b each comprise a longitudinally-extending upper concave portion 55a, 55b and lower concave portion 56a, 56b which meet at a longitudinally-extending ridge 57a, 57b.

The concave portions are spaced from the upper surface 3' and lower surface 53 by opposing convex portions 58a, 58a', 58b, 58b' such that the transverse cross-section through the substrate 2‴ is a modified obround where the opposing transverse surfaces 54a, 54b each take the form of a curly brace/bracket i.e. "{" and "}".

The length of the substrate 2‴ (between the upstream end face 59 and downstream end face 60) is around 12 mm long. The width of the substrate 2‴ (between opposing transverse surfaces 54a, 54b) may be around 12 mm. The depth of the substrate 2‴ (between the upper and lower surfaces) may be around 6 mm. Thus the aspect ratio of the length to width is 1:1, the length to depth is 1:0.5 and width to depth is 1:0.5.

The substrate 2‴ is formed of cast leaf slurry recon tobacco. It may alternatively be formed as extruded tobacco e.g. with added flavouring.

The consumable further comprises a planar filter 4". The filter 4' comprises a substantially planar upper surface 61 and a substantially planar lower surface 62 equally spaced by opposing longitudinally-extending transverse surfaces 63a, 63b.

The opposing transverse surfaces 63a, 63b each comprise a substantially convex surface (a semi-circular surface) such that the planar filter 4" has a substantially obround transverse cross section i.e. the filter 4" is an obround cylindrical filter.

The filter 4" has greater width and length than depth. The length is around 22mm and the width is around 12 mm. The depth is around 6mm. Thus the aspect ratio of the length to width is 1 :0.6, length to depth is 1:0.3 and width to depth 1 :0.5. The width to depth aspect ratio is the same for the filter as for the substrate.

The filter 4" has a hollow bore 64. The hollow bore 64 extends from the upstream longitudinal end face 5' of the filter 4" to the downstream longitudinal end face 6' of the filter 4".

The hollow bore 64 has an obround transverse cross sectional area. The bore 64 has a uniform transverse cross-sectional area. The bore is 64 dimensioned such that there is a thickness of filter material of around 1.5mm from the bore to the upper lower surfaces 61, 62 and the opposing transverse surfaces 63a, 63b.

The upstream longitudinal end face 5' of the filter 4" faces and abuts the downstream longitudinal end face 65 of the substrate 2‴.

The filter 4" is comprised of cellulose acetate or polypropylene tow. The filter 4" is circumscribed with a paper plug wrap (not shown).

The substrate 2‴ and filter 4" are contained within a rigid bagasse housing 16'.

The housing 16' comprises upper and lower walls 22', 23' (see Figure 16) spaced by opposing longitudinally-extending transverse surfaces 24a', 24b'. The housing 16' has a wall thickness in the range of around 0.8 mm.

The upper and lower walls 22', 23' are substantially planar and equally spaced by the transverse surfaces 24a', 24b', (i.e. the upper and lower walls 22', 23' are parallel to one another).

The opposing transverse surfaces 24a', 24b' each comprise upper and lower concave portions 66a, 66a', 66b, 66b' which meet at a longitudinally-extending ridge 67.

The concave portions 66a, 66a', 66b, 66b' are spaced from the upper and lower surfaces by opposing convex portions 68a, 68a', 68b, 68b' such that the transverse cross-section of the housing 16' is a modified obround.

The chamber within and defined by the inner surfaces of the housing walls 22', 23', 24a', 24b' is a modified obround cylindrical chamber, i.e. the transverse cross-section of the chamber within the housing 16' matches the transverse cross section of the substrate 2‴.

The housing 16' may have a length of around 42 mm, a height of around 6 mm and a width of around 15mm. Thus the aspect ratio of the length to width is 1:0.4, length to depth is 1:0.1 and width to depth 1:0.4.

The housing 16' has open upstream longitudinal end which is sealed by an end wall in the form of a pierceable membrane 29 in the form of a metallic foil or a plastic membrane. This pierceable membrane 29 has the same shape as the housing 16'. The pierceable membrane 29 obscures the substrate 2‴ from view and retains the substrate 2‴ within the housing 16'. To permit airflow through the substrate 2‴, the pierceable membrane 29 comprises two apertures 72a, 72b that are laterally spaced either side of a centre of the membrane 29 so as to be proximate the longitudinally-extending ridges 67 of the housing 16'. The spacing of these apertures 72a, 72b is such that, when the membrane 29 is pierced by a heating element (the profile of which is indicated by dashed lines), the apertures 72a, 72b are laterally spaced from the heating element.

The downstream longitudinal end of the housing 16' is shown in Figure 16. The downstream longitudinal end wall 21' conceals the filter 4" from view by the user.

Although the downstream longitudinal end wall 21' comprises a mouthpiece aperture 69, this is small enough (with a maximum depth of 0.6 mm and a width of 7.3 mm) that visual inspection of the filter 4" is significantly impeded.

The downstream longitudinal end wall 21', the upper wall 22', lower wall 23' and transverse side walls 24a', 24b' are coated with a hydrophobic coating or textured to provide a hydrophobic surface. In other embodiments, the downstream longitudinal end wall 21', the upper wall 22', lower wall 23' and transverse side walls 24a', 24b' comprise a layer of bagasse that has a lower porosity (e.g. around 5%) than the bagasse used to form the housing.

The consumable 1ʺʺ is heated in a heat not burn device. The device may comprise a heating element e.g. a planar heating element, for penetrating the substrate 2‴ through the upstream longitudinal end face 59. For example, the device may be as described in relation to figures 9-11.

In other embodiments, the device may comprise one or more (e.g. two) external heating elements e.g. planar external heating elements for abutment against and heating of the substrate through the upper and lower walls 22', 23' of the housing 16'.

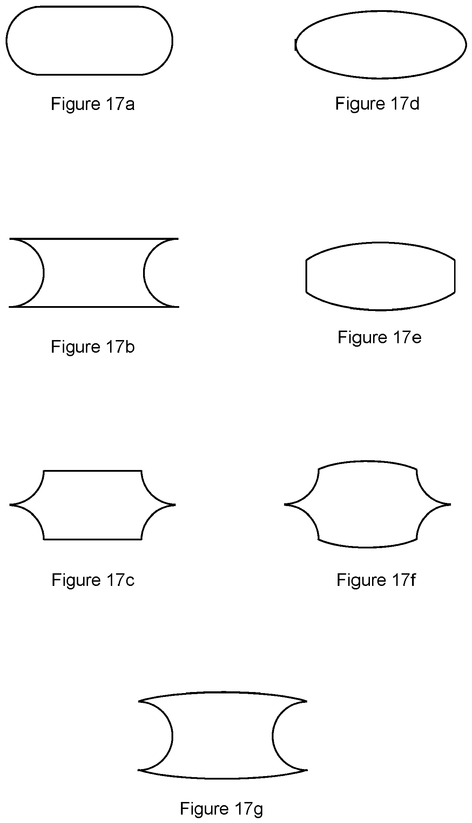

Figures 17a-17g shows various alternative transverse cross sections of the filter and/or substrate. Although they are shown without a hollow core recess, they could each comprise a hollow core recess which could have the same or different transverse cross section.

Figure 17a shows a filter or substrate or housing with planar upper and lower surfaces and convex (semi-circular) transverse surfaces such that the filter or substrate or housing has an obround transverse cross-section.

Figure 17b shows a filter or substrate or housing with planar upper and lower surfaces and concave (semi-circular) transverse surfaces.

Figure 17c shows a filter or substrate or housing which is similar to the substrate shown in Figure 15 except that there are no convex portions joining the upper and lower surfaces and the concave portions.

Figure 17d shows a filter or substrate or housing which has an oval transverse cross-sectional area.

Figure 17e shows a filter or substrate or housing with curved (convex) upper and lower surfaces and planar transverse surfaces such that the substrate has a truncated oval transverse cross-sectional area.

Figure 17f shows a filter or substrate or housing the same as Figure 17c except with curved (convex) upper and lower surfaces.

Figure 17g shows a filter or substrate or housing the same as Figure 17b except with curved (convex) upper and lower surfaces.

Although not shown, any of the filters show in Figure 17a-17g could have at least one and preferably a plurality of inwardly-extending air flow paths that may be axially aligned and evenly spaced around the perimeter of the filter.

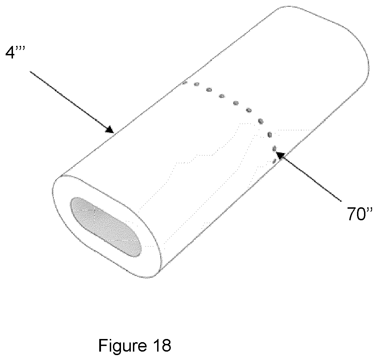

For example, Figure 18 shows a hollow bore filter 4‴ similar to Figure 17a with a plurality of axially-aligned air flow path openings 70" provided equally spaced around the perimeter of the filter i.e. extending inwardly from all of the upper, lower and transverse surfaces into the filer. The openings are proximal the midpoint of the axial length of the filter. The filter 4‴ could be used in the consumable shown in Figures 15 and 16.

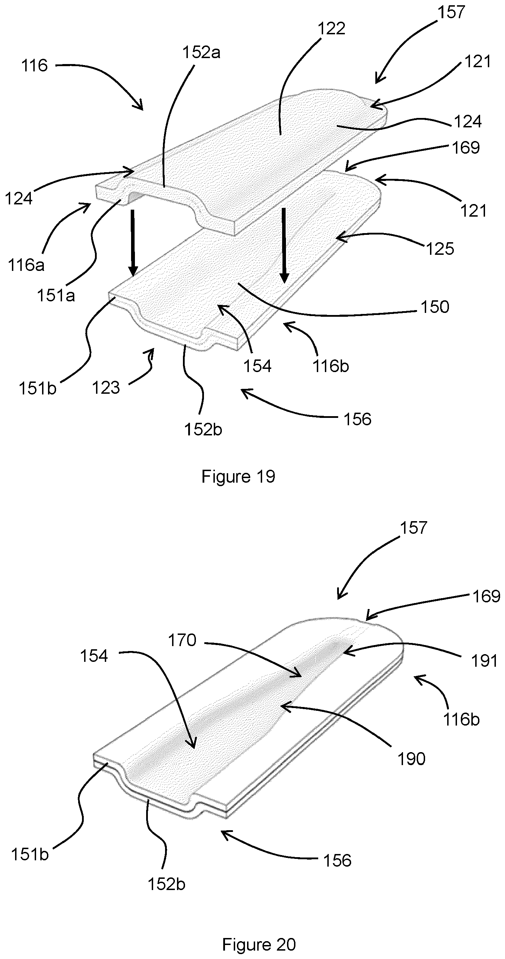

Figure 19 shows an expanded view of a housing 116 of a consumable. The housing 116 may be shaped as described with reference to any of Figures 17a-h. In the example shown in Figure 19, the transverse cross-sectional shape of the housing 116 is a modified obround, as described with reference to Figure 17c.

The housing 116 comprises an upper wall 122 and a lower wall 123 spaced by opposing transverse walls 124. The housing 116 also comprises a downstream longitudinal end wall 121 having a downstream aperture 169 for the flow of aerosol therethrough. The opposing transverse walls 124 and the downstream longitudinal end wall 121 comprise a longitudinally extending junction 125 such that the housing can be opened to expose a chamber 150 within (Figure 19 shows the housing 116 in an open position).

In Figure 19, the housing 116 is split into a pair of housing sections 116a, 116b, which are attached to one another to form the housing of the consumable (as shown by the arrows in Figure 19). The pair of housing sections 116a, 116b may be attached to one another by an adhesive, such as a biodegradable glue, for example.

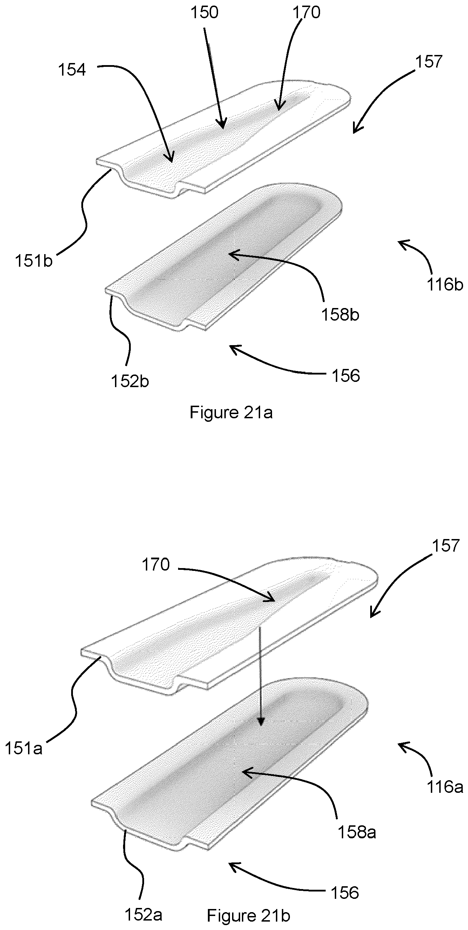

Only one of the housing sections 116b is shown in Figures 20, 21a and 21b. Each of the pair of housing sections 116a, 116b comprises an inner sleeve part 151a, 151b, and an outer housing part 152a, 152b. When the pair of housing sections 116a, 116b are brought together to form the housing of the consumable (as in Figure 19), the pair of inner sleeve parts 151a, 151b together form an inner sleeve, and the pair of outer housing parts 152a, 152b together form the walls of the housing. The walls of the housing enclose the inner sleeve.

Each inner sleeve part 151a, 151b may be attached to its respective outer housing part 152a, 152b by an adhesive such as biodegradable glue. This is illustrated by the arrow in Figure 21b.

The inner sleeve and the walls of the housing may be formed from the same material, preferably bagasse pulp.

The inner sleeve defines a chamber 150 within the housing 116. Although not shown in Figures 19 - 21b, a substrate as described with reference to any of Figures 2 and 17a-h may be enclosed within an upstream portion of the chamber 150, and therefore an upstream portion of the inner sleeve and an upstream portion 156 of the housing 116. The substrate is affixed within the upstream portion of the inner sleeve with biodegradable glue. The upstream portion of the inner sleeve has a transverse cross-sectional shape matching the transverse cross-sectional shape of the housing 116. Specifically, the transverse cross-sectional shape of the upstream portion of the inner sleeve is a modified obround.

The inner sleeve lines the walls of the housing 116. The upstream portion of the inner sleeve may conform to the shape of the walls of the upstream portion 156 of the housing.

A downstream chimney portion 170 of the chamber 150 is defined by a downstream portion of the inner sleeve when the pair of inner sleeve parts 151a, 151b are attached to one another. The chimney portion 170 extends from a downstream portion of the substrate to the downstream aperture 169. In the downstream portion of the housing 116, the inner sleeve is not contiguous with the housing 116, and the inner sleeve is transversely spaced from the transverse walls 124 of the housing 116.

As best shown in Figure 20, the transverse cross-sectional area of the chimney portion 170 reduces towards the downstream aperture 169 to direct aerosol formed at the substrate to the downstream aperture 169 and therefore to the mouth of a user. A transverse cross-sectional area of an upstream end 190 of the chimney portion 170 is greater than a cross-sectional area of a downstream end 191 of the chimney portion 170. The upstream end 190 of the chimney portion 170 is adjacent to a downstream end of the substrate.

The depth of the chimney portion 170 is substantially constant along the length of the chimney portion 170. The width of the chimney portion 170 reduces continuously from the upstream end 190 of the chimney portion 170 towards the downstream aperture 169. Transverse walls of the chimney portion 170 comprise a substantially convex surface facing the chimney portion 170.

As shown in Figure 20, each of the transverse walls of the chimney portion 190 are non-linear curved walls, which deflect inwardly towards the opposing transverse wall of the chimney portion 190 before extending to the downstream aperture 169.

A method of making the housing 116 according to the first embodiment will now be described with reference to Figures 19-21b.

Firstly, each of the pair of insert parts 151a, 151b is mounted to a respective outer housing part 152a, 152b to form a respective housing section 116a, 116b. This step is illustrated by the arrow in Figure 21b. Each of the outer housing parts 152a, 152b comprises a respective indentation 158a, 158b, into which the respective insert part 151a, 151b is mounted. Each insert part 151a, 151b is glued to the respective outer housing part 152a, 152b using a biodegradable adhesive. An example of a resulting housing section 116b is shown in Figure 20. Each housing section 116a, 116b may form a half of the housing 116 of the consumable.

Next, the aerosol forming substrate (as described with reference to any of Figures 2 and 17a-h) is mounted to either of the two housing sections 116a, 116b (not shown in the figures) at an upstream portion 156 of the housing section 116a, 116b.

Finally, the housing sections are attached together (by a biodegradable glue) around their periphery, thereby forming the chamber 150 therein. This is illustrated by the arrows shown in Figure 19. The resultant housing 116 encloses the substrate in the upstream portion of the chamber 150, and defines the tapered chimney portion extending from the substrate to the mouthpiece aperture 169.

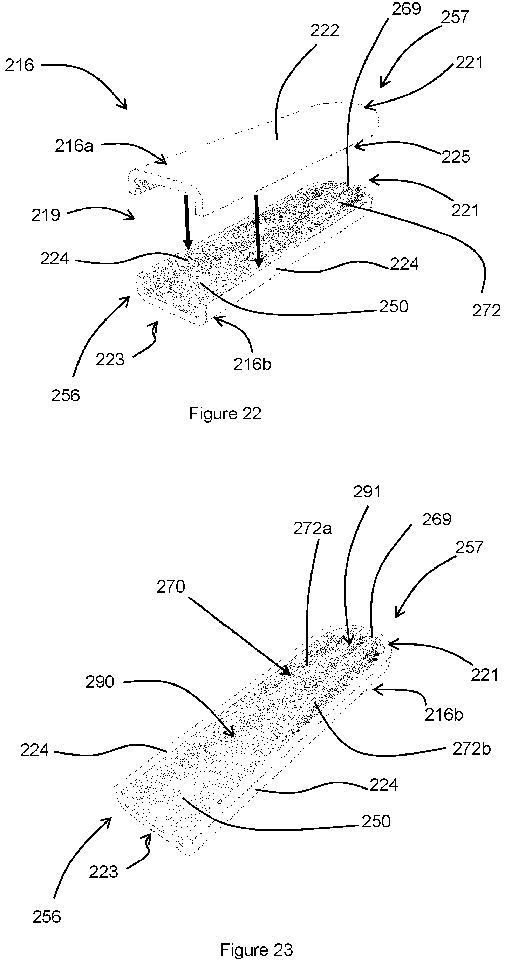

Figure 22 shows an expanded view of a housing 216 for a consumable. The housing 216 may be shaped as described with reference to any of Figures 17a-h. In the example shown in Figure 22, the transverse cross-sectional shape of the housing 216 is generally rectangular.

The housing comprises an upper outer 222 and a lower wall 223 spaced by opposing transverse walls 224. The housing 216 also comprises a downstream longitudinal end wall 221 having a downstream aperture 269 for the flow of aerosol therethrough. The opposing transverse walls 224 and the downstream longitudinal end wall 221 comprise a longitudinally extending junction 225 such that the housing can be opened to expose a chamber 250 within (Figure 22 shows the housing 216 in an open position).

The upper and lower walls 222, 223 are substantially planar and are equally spaced by the transverse walls 224, such that the housing 216 is a substantially planar housing. The opposing transverse walls 224 are also planar and substantially parallel to one another, and substantially perpendicular to the upper and lower walls 222, 223. The downstream longitudinal end wall 221 is rounded (i.e. convex). An opposing open upstream longitudinal end face 219 has a substantially rectangular transverse cross-section.

In Figure 22, the housing 216 is split into a pair of housing sections 216a, 216b, which are attached to one another to form the housing 216 (as shown by the arrows in Figure 22). The pair of housing sections 216a, 216b may be attached to one another by an adhesive, such as biodegradable glue. Figure 23 shows one of the pair of housing sections 216b.

Although not shown in Figures 22 and 23, a substrate as described with reference to any of Figures 2 and 17a-h may be enclosed within an upstream portion of the chamber 250, and therefore an upstream portion 256 of the housing 216. The substrate is affixed within the upstream portion of the chamber 250 by a biodegradable glue.

As shown in Figures 22 and 23, the housing 216 further comprises webbing 272 defining a downstream chimney portion 270 of the chamber 250. The webbing 272 is formed in a downstream portion 257 of the housing 216. Specifically, the webbing 272 extends from a downstream portion of the substrate to the mouthpiece aperture 269.

The webbing 272 comprises two curved webs 272a and 272b. Each web 272a, 272b extends from a respective transverse wall 224 of the housing 216 adjacent to the substrate, transversely inwards towards the opposing web 272a, 272b and longitudinally to the downstream aperture 269. The webs 272a, 272b are bow-shaped.

The transverse cross-sectional area of the chimney portion 270 in Figure 23 reduces towards the downstream aperture 269 to direct aerosol formed at the substrate to the downstream aperture 269 and therefore to the mouth of a user. A transverse cross-sectional area of an upstream end 290 of the chimney portion 270 is greater than a cross-sectional area of a downstream end 291 of the chimney portion 270.

The transverse cross-sectional area of the chimney portion 270 has a generally rectangular cross-section along its entire length. It is defined by the two longitudinally-extending webs 272a, 272b, and the upper and lower walls 222, 223 of the housing 216. Therefore, the webs 272a, 272b are spaced from one another by a greater distance at the upstream end 290 of the chimney portion 270 than at the downstream end 291 of the chimney portion 270. Thus, the width of the chimney portion 270 reduces towards the downstream aperture 269, but the depth of the chimney portion 270 remains substantially constant along its length.

In the example shown in Figures 22 and 23, the webbing 272 is integrally formed with the walls of the housing 216. The webs 272a, 272b extend from a respective transverse wall 224 of the housing 216 at a position adjacent to a downstream end of the substrate, transversely inwards into the chamber 250, and longitudinally to the mouthpiece aperture 269. The webs 272a, 272b also extends between the upper and lower walls 222, 223 such that the upper and lower walls of the housing are spaced by both the webs 272a, 72b and the transverse walls 224 of the housing 216. The webs 272a, 272b extend between the upper and lower walls 222, 223 of the housing 216 in a direction substantially perpendicular to the upper and lower walls. Accordingly, when the pair of housing sections 216a, 216b are brought together (as illustrated in Figure 22), the webs 272a, 272b and the upper and lower walls 222, 223 of the housing define the downstream chimney portion 270.

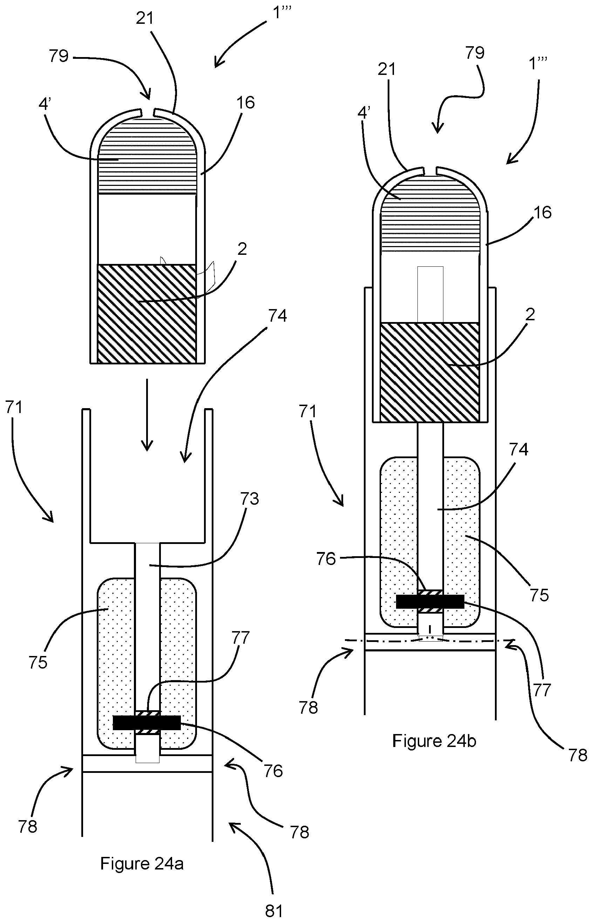

Figures 24a and 24b show a smoking substitute system which includes a consumable 1"'. The illustrated consumable 1‴ is the same as that shown in figures 4a and 4b. The planar substrate 2 formed of a freeze-dried tobacco mixture such that the mixture has a porosity of between 20% and 70%.

The substrate 2 is a freeze-dried (moulded) tobacco substrate 2. Whilst not apparent from the Figure 4a-4b, the mixture has a porosity of between 20% and 70%.

The system also comprises a vapour generating article 71, which is integral with the main body of a smoking substitute device 81 (i.e. an e-cigarette device). The vapour generating article 71 comprises a recess 73, into which the consumable 1‴ can be received and engaged by way of an interference fit. Figure 24b shows the consumable 1‴ in an engaged position, whilst Figure 24a shows the consumable 1‴ in a disengaged position.

The vapour generating article 71 further comprises a passage 74 for fluid flow therethrough. The passage 74 extends through the vapour generating article, between openings that define an inlet and an outlet of the passage 74. The passage 74 is surrounded and defined by a tank 75 containing a vaporisable liquid (e.g. an e-liquid). The e-liquid may, for example, include propylene glycol and/or vegetable glycerine.

This e-liquid can be vaporised by a vaporiser of the vapour generating article 71. The vaporiser comprises a wick 76 and a heater 77. The wick 76 is formed of a porous material and extends across the passage 75 such that opposing ends of the wick 76 extend into the tank 75, whilst a central portion of the wick 76 is exposed to fluid flow in the passage 75. In this way, e-liquid stored in the tank 75 is drawn from the ends of the wick 76 to the central portion of the wick 76 by capillary action.

The heater 77 comprises a heating filament that is wound about the wick 76. The heater 77 is connected electrically to a power source of the device 81 (not shown in the present figures). When power is supplied to the heating filament of the heater 77, the heating filament rises in temperature so as to heat the wick 76 and the e-liquid in the wick 76. When heated, the e-liquid forms a vapour, which is entrained in an airflow flowing through the passage 73.

The passage 73 of the vapour generating article 71 is fluidly connected between inlets 78 of the vapour generating article 71 and an outlet 79 formed in the longitudinal end wall 21 of the consumable 1 "'. Thus, as may be appreciated from Figure 24b in particular, when the consumable 1‴ is engaged with the vapour generating article 71, a user can draw air through the vapour generating article 71 and the consumable 1‴ (from the inlets 78). That is, a user can inhale through the mouthpiece outlet 79, which draws air through the passage 73 and subsequently through the consumable 1 "'.

As the air passes across the wick 76, vapour (produced by heating e-liquid) is entrained in the air. This vapour is carried through the passage 73 and then through the freeze-dried (moulded) tobacco substrate 2 of the consumable 1 "'. The porous nature of the substrate 2 allows the vapour to pass therethrough. As the vapour passes through the substrate 2 it rehydrates the substrate 2, which causes material (such as nicotine) of the substrate 2 to become entrained in the airflow/vapour. The vapour may subsequently cool to form an aerosol and pass through the filter 4' before being discharged from the consumable 1‴ through the outlet 79 (i.e. so as to be inhaled by a user).

The invention has been described in conjunction with the exemplary embodiments described above. The exemplary embodiments of the invention set forth above are considered to be illustrative and not limiting. Various changes to the described embodiments may be made without departing from the scope of the invention.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

## Claims

1. A heat not burn (HNB) consumable (1ʺʺ) comprising an aerosol-forming substrate (2‴) and a downstream filter (4") having upper and lower surfaces (61, 62) spaced by opposing longitudinally-extending transverse surfaces (63a, 63b) wherein the filter (4") has a greater width than depth and wherein the filter (4") comprises at least one inwardly-extending air flow path (70") extending from at least one of the upper, lower or transverse surfaces (61, 62, 63a, 63b) towards an axial centre of the filter (4"), **characterised in that** the at least one air flow path (70") is defined by a channel having an opening (70) provided on one of the surfaces (61, 62, 63a, 63b).

2. A consumable (1 "") according to claim 1 wherein the upper and lower surfaces (61, 62) of the filter (4") are substantially planar and equally spaced by planar transverse surfaces (63a, 63b) such that the filter (4") is a cuboid filter.

3. A consumable (1ʺʺ) according to claim 1 wherein at least one of the upper, lower and transverse surfaces (61, 62, 63a, 63b) is a curved or rounded surface or comprises a curved or rounded surface portion.

4. A consumable (1ʺʺ) according to claim 3 wherein the filter (4") has convex or concave opposing transverse surfaces (63a, 63b).

5. A consumable (1ʺʺ) according to claim 4 wherein each transverse surface (63a, 63b) comprises longitudinally-extending upper and lower concave portions which meet at a longitudinally-extending ridge.

6. A consumable (1ʺʺ) according to any one of claims 3 to 5 wherein one or both of the upper/lower surfaces (61, 62) is a convex rounded surface.

7. A consumable (1"") according to any one of the preceding claims comprising a plurality of inwardly-extending air flow paths (70").

8. A consumable (1"") according to claim 7 wherein the plurality of air flow paths (70") are equally spaced around the perimeter of the filter (4").

9. A consumable (1"") according to any one of the preceding claims wherein the filter (4") comprises a hollow bore (64).

10. A consumable (1ʺʺ) according to any one of the preceding claims comprising a housing (16') having a mouthpiece (69) wherein the filter (4") is substantially concealed from view by a downstream longitudinal end face (21') of the housing (16').

11. A heat not burn (HNB) system comprising:
a heat not burn consumable (1ʺʺ) according to any one of claims 1 to 10; and
a device (34) comprising at least one heating element (28).

12. A system according to claim 11 wherein the device (28) comprises a device housing (34) having a cavity (35) for receiving the consumable (1ʺʺ) wherein the at least one heating element (28) projects into or surrounds the cavity (35).

13. A method of using a heat not burn system according to claim 11 or 12, the method comprising:
inserting the consumable (1ʺʺ) into the device (28); and
heating the consumable (1"") using the heating element (28).

## Patentansprüche

1. Erhitzen-anstatt-Verbrennen-(HNB-)Verbrauchsware (1""), umfassend ein aerosolbildendes Substrat (2‴) und ein stromabwärtiges Filter (4") mit einer oberen und unteren Fläche (61, 62), die durch entgegengesetzte, sich der Länge nach erstreckende Querflächen (63a, 63b) voneinander beabstandet sind, wobei die Breite des Filters (4") größer ist als seine Tiefe und wobei das Filter (4") zumindest einen sich nach innen erstreckenden Luftströmungspfad (70") aufweist, der sich von zumindest einer der oberen, unteren oder Querflächen (61, 62, 63a, 63b) in Richtung einer axialen Mitte des Filters (4") erstreckt, **dadurch gekennzeichnet, dass** der zumindest eine Luftströmungspfad (70") durch einen Kanal definiert ist, der eine auf einer der Flächen (61, 62, 63a, 63b) bereitgestellte Öffnung (70) aufweist.

2. Verbrauchsware (1ʺʺ) nach Anspruch 1, wobei die obere und die untere Fläche (61, 62) des Filters (4") im Wesentlichen planar und durch planare Querflächen (63a, 63b) gleichmäßig voneinander beabstandet sind, sodass das Filter (4") ein quaderförmiges Filter ist.

3. Verbrauchsware (1ʺʺ) nach Anspruch 1, wobei zumindest eine der oberen, unteren und Querflächen (61, 62, 63a, 63b) eine gekrümmte oder gerundete Fläche ist oder einen gekrümmten oder gerundeten Flächenabschnitt umfasst.

4. Verbrauchsware (1ʺʺ) nach Anspruch 3, wobei das Filter (4") konvexe oder konkave entgegengesetzte Querflächen (63a, 63b) aufweist.

5. Verbrauchsware (1ʺʺ) nach Anspruch 4, wobei jede Querfläche (63a, 63b) sich der Länge nach erstreckende obere und untere konkave Abschnitte umfasst, die an einer sich der Länge nach erstreckenden Erhöhung aufeinandertreffen.

6. Verbrauchsware (1ʺʺ) nach einem der Ansprüche 3 bis 5, wobei eine oder beide der oberen/unteren Flächen (61, 62) eine konvex gerundete Fläche ist.

7. Verbrauchsware (1ʺʺ) nach einem der vorangegangenen Ansprüche, umfassend eine Vielzahl von sich nach innen erstreckenden Luftströmungspfade (70").

8. Verbrauchsware (1ʺʺ) nach Anspruch 7, wobei die Vielzahl von Luftströmungspfaden (70") um den Umfang des Filters (4") herum gleichmäßig voneinander beabstandet sind.

9. Verbrauchsware (1ʺʺ) nach einem der vorangegangenen Ansprüche, wobei das Filter (4") eine Hohlbohrung (64) umfasst.

10. Verbrauchsware (1‴) nach einem der vorangegangenen Ansprüche, umfassend ein Gehäuse (16') mit einem Mundstück (69), wobei das Filter (4") durch eine stromabwärtige längliche Endfläche (21') des Gehäuses (16') im Wesentlichen nicht sichtbar ist.

11. Erhitzen-anstatt-Verbrennen-(HNB-)System, umfassend:
eine Erhitzen-anstatt-Verbrennen-(HNB-)Verbrauchsware (1ʺʺ) nach einem der Ansprüche 1 bis 10; und
eine Vorrichtung (34), die zumindest ein Heizelement (28) umfasst.

12. System nach Anspruch 11, wobei die Vorrichtung (28) ein Vorrichtungsgehäuse (34) mit einem Hohlraum (35) zum Aufnehmen der Verbrauchsware (1ʺʺ) umfasst, wobei das zumindest eine Heizelement (28) in den Hohlraum (35) hineinragt oder diesen umgibt.

13. Verfahren zur Verwendung eines Erhitzen-anstatt-Verbrennen-(HNB-)Systems nach Anspruch 11 oder 12, wobei das Verfahren Folgendes umfasst:
Einsetzen der Verbrauchsware (1ʺʺ) in die Vorrichtung (28); und
Erhitzen der Verbrauchsware (1ʺʺ) unter Verwendung des Heizelements (28).

## Revendications

1. Produit consommable chauffé sans combustion (HNB) (1ʺʺ) comprenant un substrat de formation d'aérosol (2''') et un filtre aval (4") présentant des surfaces supérieure et inférieure (61, 62) espacées par des surfaces transversales opposées s'étendant longitudinalement (63a, 63b), dans lequel le filtre (4") présente une largeur supérieure à sa profondeur et dans lequel le filtre (4ʺ) comprend au moins un trajet d'écoulement d'air s'étendant vers l'intérieur (70") s'étendant d'au moins l'une des surfaces supérieure, inférieure ou transversales (61, 62, 63a, 63b) vers un centre axial du filtre (4"), **caractérisé en ce que** le au moins un trajet d'écoulement d'air (70") est défini par un canal présentant une ouverture (70) prévue sur l'une des surfaces (61, 62, 63a, 63b).

2. Produit consommable (1ʺʺ) selon la revendication 1, dans lequel les surfaces supérieure et inférieure (61, 62) du filtre (4") sont sensiblement planes et espacées de manière égale par des surfaces transversales planes (63a, 63b) de telle sorte que le filtre (4ʺ) soit un filtre cuboïde.

3. Produit consommable (1ʺʺ) selon la revendication 1, dans lequel au moins l'une des surfaces supérieure, inférieure et transversales (61, 62, 63a, 63b) est une surface incurvée ou arrondie ou comprend une partie de surface incurvée ou arrondie.

4. Produit consommable (1ʺʺ) selon la revendication 3, dans lequel le filtre (4ʺ) présente des surfaces transversales opposées convexes ou concaves (63a, 63b).

5. Produit consommable (1ʺʺ) selon la revendication 4, dans lequel chaque surface transversale (63a, 63b) comprend des parties concaves supérieure et inférieure s'étendant longitudinalement qui se rejoignent au niveau d'une arête s'étendant longitudinalement.

6. Produit consommable (1ʺʺ) selon l'une quelconque des revendications 3 à 5, dans lequel l'une des surfaces supérieure/inférieure (61, 62), ou les deux, est une surface arrondie convexe.

7. Produit consommable (1ʺʺ) selon l'une quelconque des revendications précédentes, comprenant une pluralité de trajets d'écoulement d'air s'étendant vers l'intérieur (70").

8. Produit consommable (1ʺʺ) selon la revendication 7, dans lequel la pluralité de trajets d'écoulement d'air (70") sont espacés de manière égale autour du périmètre du filtre (4").

9. Produit consommable (1ʺʺ) selon l'une quelconque des revendications précédentes lequel le filtre (4") comprend un alésage creux (64).

10. Produit consommable (1ʺʺ) selon l'une quelconque des revendications précédentes, comprenant un boîtier (16') présentant un embout buccal (69), dans lequel le filtre (4") est sensiblement caché à la vue par une face d'extrémité longitudinale aval (21') du boîtier (16').

11. Système de chauffage sans combustion (HNB), comprenant :
un produit consommable chauffé sans combustion (1ʺʺ) selon l'une quelconque des revendications 1 à 10 ; et
un dispositif (34) comprenant au moins un élément chauffant (28).

12. Système selon la revendication 11, dans lequel le dispositif (28) comprend un boîtier de dispositif (34) présentant une cavité (35) destinée à recevoir le produit consommable (1ʺʺ), dans lequel le au moins un élément chauffant (28) fait saillie dans ou entoure la cavité (35).

13. Procédé d'utilisation d'un système de chauffage sans combustion selon la revendication 11 ou 12, le procédé comprenant les étapes consistant à :
insérer le produit consommable (1ʺʺ) dans le dispositif (28) ; et
chauffer le produit consommable (1'''') à l'aide de l'élément chauffant(28).
